# EUROPEAN PATENT APPLICATION

(11) **EP 1 731 524 A1**
(43) Date of publication of application: **13.12.2006**
(21) Application number: 05728907.6
(22) Date of filing: 30.03.2005
(51) Int. Cl.: C07H 15/203, A61K 31/7034, A61P 3/10

(54) **PHENOL DERIVATIVE, MEDICINAL COMPOSITION CONTAINING THE SAME, AND MEDICINAL USE THEREOF**

(30) Priority: 31.03.2004 JP 2004101893
(71) Applicant: Kissei Pharmaceutical Co., Ltd., Matsumoto-shi Nagano 399-8710 (JP)
(72) Inventor: FUJIKURA, Hideki, Kissei Pharmaceutical Co., Ltd., Minamiazumi-gun, Nagano 399-8304 (JP); FUSHIMI, Nobuhiko, Kissei Pharmaceutical Co., Ltd., Minamiazumi-gun, Nagano 399-8304 (JP); ISAJI, Masayuki, Kissei Pharmaceutical Co., Ltd., Minamiazumi-gun, Nagano 399-8304 (JP)
(74) Representative: Hayes, Adrian Chetwynd
(86) International application number: PCT/JP2005/006702
(87) International publication number: WO 2005/095429

(57) **Abstract**

The present invention provides phenol derivatives represented by the following general formula or pharmaceutically acceptable salts thereof, or prodrugs thereof, which exhibit an inhibitory activity in human SGLT and are useful as agents for the prevention or treatment of a disease associated with hyperglycemia such as diabetes, postprandial hyperglycemia, impaired glucose tolerance, diabetic complications, obesity or the like, and pharmaceutical compositions comprising the same, and pharmaceutical uses thereof. In the chemical structure, R¹ and R² represent H, OH, NH₂, etc.; R³ and R⁴ represent H, OH, a halogen atom, an optionally substituted alkyl group, etc.; ring A represents an aryl group or a heteroaryl group; G represents a group represented by the following general formula (G); E¹ represents H or F; and E² represents H, F, or a methyl group, etc.

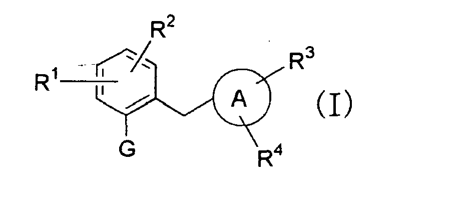

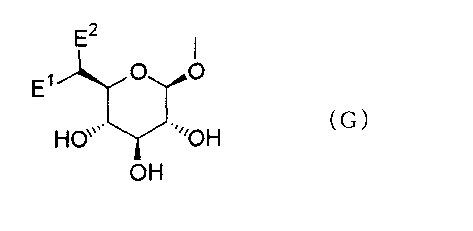

## Description

### Technical Field

The present invention relates to phenol derivatives, pharmaceutically acceptable salts thereof or prodrugs thereof, which are useful as medicaments, pharmaceutical compositions comprising the same and pharmaceutical uses thereof.

More particularly, the present invention relates to phenol derivatives having an inhibitory activity in human SGLT, pharmaceutically acceptable salts thereof or prodrugs thereof which are useful as agents for the prevention or treatment of a disease associated with hyperglycemia such as diabetes, impaired glucose tolerance, diabetic complications or obesity, pharmaceutical compositions comprising the same and pharmaceutical uses thereof.

### Background Art

Diabetes is one of lifestyle-related diseases with the background of change of eating habit and lack of exercise. Hence, diet and exercise therapies are performed in patients with diabetes. Furthermore, when its sufficient control and continuous performance are difficult, drug treatment is simultaneously performed. In addition, it has been confirmed by large-scale clinical trial that it is necessary to practice a long-term strict control of blood sugar level so as to prevent patients with diabetes from occurring and advancing diabetic complications by receiving treatment (for example, see the following References 1 and 2). Furthermore, many epidemiologic studies on impaired glucose tolerance and macroangiopathy show that impaired glucose tolerance as the boundary type is also a risk factor in macroangiopathy as well as diabetes. Thus, needs to improve postprandial hyperglycemia have been focused (for example, see the following Reference 3).

In recent years, development of various antidiabetic agents has been progressing with the background of a rapid increase of patients with diabetes. For example, Antidiabetic agents such as biguanides, sulfonylureas, insulin sensitivity enhancers, α-glucosidase inhibitors and the like have been employed. However, biguanides and sulfonylureas show occasionally adverse effects such as lactic acidosis and hypoglycemia, respectively. Insulin sensitivity enhancers show occasionally adverse effects such as edema, and are concerned for advancing obesity. In addition, α-glucosidase inhibitors, which delay carbohydrate digestion and absorption at the small intestine, are used to improve postprandial hyperglycemia. It has been also reported that acarbose, one of α-glucosidase inhibitors, has an effect of preventing or delaying the incidence of diabetes by applying to patients with impaired glucose tolerance (for example, see the following Reference 4). However, since α-glucosidase inhibitors do not affect elevated glucose levels by ingesting a monosaccharide of glucose (for example, see the following Reference 5), with recently changing compositions of sugars in meals, a wider range of activities inhibiting carbohydrate absorption has been desired.

In recent years, research and development of new type antidiabetic agents have been progressing, which promote urinary glucose excretion and lower blood glucose level by preventing reabsorption of excess glucose at the kidney (for example, see the following Reference 6). In addition, it is reported that SGLT2 (sodium-dependent glucose transporter 2) is present in the S1 segment of the kidney's proximal tubule and participates mainly in reabsorption of glucose filtrated through glomerular (for example, see the following Reference 7). Accordingly, inhibiting a human SGLT2 activity prevents reabsorption of excess glucose at the kidney, subsequently promotes excreting excess glucose though the urine, and normalizes blood glucose level. In addition, since such agents for promoting the excretion of urinary glucose excrete excess glucose though the urine and consequently the glucose accumulation in the body is decreased, they are also expected to have a preventing or alleviating effect on obesity and a diuretic effect. Furthermore, the agents are considered to be useful for various related diseases which occur accompanying the progress of diabetes or obesity due to hyperglycemia.

Furthermore, it has been known that SGLT1, sodium-dependent glucose transporter 1, exists in the small intestine which controls carbohydrate absorption. It has been also reported that insufficiency of glucose and galactose absorption arises in patients with dysfunction due to congenital abnormalities of human SGLT1 (for example, see the following References 8-10). In addition, it has been confirmed that SGLT1 is involved in glucose and galactose absorption (for example, see the following References 11 and 12). Furthermore, it is confirmed that mRNA and protein of SGLT1 increase and absorption of glucoses are accelerated in OLETF rats and rats with streptozotocin-induced diabetic symptoms (for example, see the following References 13 and 14). Generally in patients with diabetes, carbohydrate digestion and absorption are increased. For example, it is confirmed that mRNA and protein of SGLT1 are highly increased in the human small intestine (for example, see the following Reference 15). Therefore, blocking a human SGLT1 activity inhibits absorption of carbohydrates such as glucose at the small intestine, subsequently can prevent increase of blood sugar level. Especially, it is considered that delaying glucose absorption based on the above mentioned mechanism is effective to normalize postprandial hyperglycemia.

Therefore, fast development of antidiabetic agents with novel action mechanism, which have an inhibitory activity in human SGLT, has been desired to improve or solve the above-mentioned problems.

Phenol derivatives provided in the present invention are entirely novel compounds. It has not ever been reported that these derivatives have an inhibitory activities in SGLT1 and/or SGLT2 and inhibit absorption of glucose and galactose at the small intestine, or are useful as agents to inhibit reabsorption of excess glucose at the kidney.
Reference 1: The Diabetes Control and Complications Trial Research Group, N. Engl. J. Med., 1993.9, Vol.329, No.14, pp.977-986;
Reference 2: UK Prospective Diabetes StudyGroup, Lancet, 1998.9, Vol.352, No.9131, pp.837-853;
Reference 3: Makoto TOMINAGA, Endocrinology & Diabetology, 2001.11, Vol.13, No.5, pp.534-542;
Reference 4: Jean-Louis Chiasson and 5 persons, Lancet, 2002.6, Vol.359, No.9323, pp.2072-2077;
Reference 5: Hiroyuki ODAKA and 3 persons, Journal of Japanese Society of Nutrition and Food Science, 1992, Vol.45, p.27;
Reference 6: Luciano Rossetti and 4 persons, J. Clin. Invest., 1987.5, Vol.79, pp.1510-1515;
Reference 7: Yoshikatsu Kanai and 4 persons, J. Clin. Invest., 1994.1, Vol.93, pp.397-404;
Reference 8: Tadao BABA and 1 person, Supplementary volume of Nippon Rinsho, Ryoikibetsu Shokogun, 1998, No.19, pp.552-554;
Reference 9: Michihiro KASAHARA and 2 persons, Saishin Igaku, 1996.1, Vol.51, No.1, pp.84-90;
Reference 10: Tomofusa TSUCHIYA and 1 person, Nippon Rinsho, 1997.8, Vol.55, No.8, pp.2131-2139;
Reference 11: Yoshikatsu KANAI, Kidney and Dialysis, 1998.12, Vol.45, extra edition, pp.232-237;
Reference 12: E. Turk and 4 persons, Nature, 1991.3, Vol.350, pp.354-356;
Reference 13: Y. Fujita and 5 persons, Diabetologia, 1998, Vol. 41, pp.1459-1466;
Reference 14: J. Dyer and 5 persons, Biochemical Society Transactions, 1997, Vol.25, p.479S;
Reference 15: J. Dyer and 4 persons, American Journal of Physiology, 2002.2, Vol.282, No.2, pp.G241-G248

### Disclosure of the Invention

The present inventors have studied earnestly to find compounds having an inhibitory activity in human SGLT. As a result, it was found that certain phenol derivatives represented by the following general formula (I) show an inhibitory activity in human SGLT1 and/or SGLT2 and are excellent agents having inhibitory activity in increase of blood glucose level or lowering blood glucose level as shown below, thereby forming the basis of the present invention.

The present invention is to provide novel compounds which show an inhibitory activity in human SGLT, pharmaceutical compositions comprising the same and pharmaceutical uses thereof.

This is, the present invention relates to
[1] a phenol derivative represented by the following general formula (I): 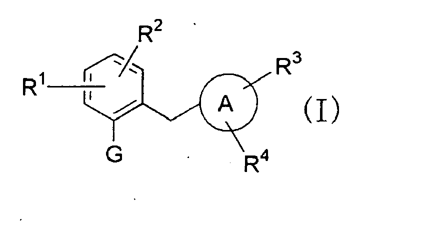 wherein
   R¹ or R² independently represents a hydrogen atom, a hydroxy group, an amino group, a halogen atom, a C₁₋₆ alkyl group, a C₁₋₆ alkoxy group, a cyano group, a carboxy group, a C₂₋₇ alkoxycarbonyl group, a carbamoyl group, a mono or di(C₁₋₆ alkyl)amino group, a halo(C₁₋₆ alkyl) group, a hydroxy(C₁₋₆ alkyl) group, a cyano (C₁₋₆ alkyl) group, a carboxy (C₁₋₆ alkyl) group, a C₂₋₇ alkoxycarbonyl (C₁₋₆ alkyl) group, a carbamoyl (C₁₋₆ alkyl) group, an amino(C₁₋₆ alkyl) group, a mono or di(C₁₋₆ alkyl)amino(C₁₋₆ alkyl) group, a halo(C₁₋₆ alkoxy) group, a hydroxy (C₁₋₆ alkoxy) group, a carboxy (C₁₋₆ alkoxy) group, a C₂₋₇ alkoxycarbonyl(C₁₋₆ alkoxy) group, a carbamoyl(C₁₋₆ alkoxy) group, an amino(C₁₋₆ alkoxy) group, a mono or di(C₁₋₆ alkyl)amino(C₁₋₆ alkoxy) group, a C₃₋₇ cycloalkyl group, C₃₋₇ cycloalkyl-O-, a C₃₋₇ cycloalkyl(C₁₋₆ alkyl) group, or C₃₋₇ cycloalkyl(C₁₋₆ alkoxy) group;
   R³ and R⁴ independently represent a hydrogen atom, a hydroxy group, a halogen atom, a C₁₋₆ alkyl group, a C₂₋₆alkenyl group, a C₂₋₆ alkynyl group, a C₁₋₆ alkoxy group, a C₂₋₆ alkenyloxy group, a C₁₋₆ alkylthio group, a C₂₋₆ alkenylthio group, a halo(C₁₋₆ alkyl) group, a halo(C₁₋₆alkoxy) group, a halo (C₁₋₆ alkylthio) group, a hydroxy (C₁₋₆ alkyl) group, a hydroxy (C₂₋₆ alkenyl) group, a hydroxy (C₁₋₆ alkoxy) group, a hydroxy (C₁₋₆ alkylthio) group, a carboxy group, a carboxy(C₁₋₆ alkyl) group, a carboxy(C₂₋₆ alkenyl) group, a carboxy(C₁₋₆ alkoxy) group, a carboxy(C₁₋₆ alkylthio) group, a C₂₋₇ alkoxycarbonyl group, a C₂₋₇ alkoxycarbonyl(C₁₋₆ alkyl) group, a C₂₋₇ alkoxycarbonyl (C₂₋₆ alkenyl) group, a C₂₋₇ alkoxycarbonyl (C₁₋₆ alkoxy) group, a C₂₋₇ alkoxycarbonyl (C₁₋₆ alkylthio) group, a C₁₋₆ alkylsulfinyl group, a C₁₋₆ alkylsulfonyl group, -U-V-W-N(R⁵)-Z, or any of the following substituents (i) to (xxviii) which may have any 1 to 3 substituents selected from the later identified substituent group α on the ring;
   (i) a C₆₋₁₀ aryl group, (ii) C₆₋₁₀ aryl-O-, (iii) C₆₋₁₀ aryl-S-, (iv) a C₆₋₁₀ aryl (C₁₋₆alkyl) group, (v) a C₆₋₁₀ aryl (C₁₋₆ alkoxy) group, (vi) a C₆₋₁₀ aryl(C₁₋₆ alkylthio) group, (vii) a heteroaryl group, (viii) heteroaryl-O-, (ix) heteroaryl-S-, (x) a heteroaryl(C₁₋₆ alkyl) group, (xi) a heteroaryl(C₁₋₆ alkoxy) group, (xii) a heteroaryl(C₁₋₆alkylthio) group, (xiii) a C₃₋₇ cycloalkyl group, (xiv) C₃₋₇ cycloalkyl-O-, (xv) C₃₋₇ cycloalkyl-S-, (xvi) a C₃₋₇ cycloalkyl (C₁₋₆ alkyl) group, (xvii) a C₃₋₇ cycloalkyl(C₁₋₆ alkoxy) group, (xviii) a C₃₋₇ cycloalkyl(C₁₋₆ alkylthio) group, (xix) a heterocycloalkyl group, (xx) heterocycloalkyl-O-, (xxi) heterocycloalkyl-S-, (xxii) a heterocycloalkyl(C₁₋₆ alkyl) group, (xxiii) a heterocycloalkyl(C₁₋₆ alkoxy) group, (xxiv) a heterocycloalkyl(C₁₋₆ alkylthio) group, (xxv) an aromatic cyclic amino group, (xxvi) an aromatic cyclic amino (C₁₋₆ alkyl) group, (xxvii) an aromatic cyclic amino(C₁₋₆ alkoxy) group or (xxviii) an aromatic cyclic amino(C₁₋₆ alkylthio) group,
   U represents -O-, -S- or a single bond and with the proviso that at least one of V and W is not a single bond when U is -O- or -S-);
   V represents a C₁₋₆ alkylene group which may have a hydroxy group, a C₂₋₆ alkenylene group or a single bond;
   W represents -CO-, -SO₂-, -C(=NH)- or a single bond;
   Z represents a hydrogen atom, a C₂₋₇ alkoxycarbonyl group, a C₆₋₁₀ aryl(C₂₋₇ alkoxycarbonyl) group, a formyl group, -R^{A}, -COR^{B}, -SO₂R^{B}, -CON(R^{C})R^{D}, -CSN(R^{C}) R^{D}, -SO₂NHR^{A} or -C(=NR^{E})N(R^{F})R^{G};
   R , R , R and R independently represent a hydrogen atom, a C₁₋₆ alkyl group which may have any 1 to 5 substituents selected from the later identified substituent group β, or any of the following substituents (xxix) to (xxxii) which may have any 1 to 3 substituents selected from the later identified substituent group α;
   (xxix) a C₆₋₁₀ aryl group, (xxx) a heteroaryl group, (xxxi) a C₃₋₇ cycloalkyl group or (xxxii) a heterocycloalkyl group
   or Z and R⁵ bind together with the neighboring nitrogen atom to form an aliphatic cyclic amino group which may have any 1 to 3 substituents selected from the later identified substituent group α;
   or R^{C} and R^{D} bind together with the neighboring nitrogen atom to form an aliphatic cyclic amino group which may have any 1 to 3 substituents selected from the later identified substituent group α;
   R^{B} represents a C₂₋₇ alkoxycarbonyl group, a C₁₋₆ alkylsulfonylamino group, a C₆₋₁₀ arylsulfonylamino group, a C₁₋₆ alkyl group which may have any 1 to 5 substituents selected from the later identified substituent group β, or any of the following substituents (xxxiii) to (xxxvi) which may have any 1 to 3 substituents selected from the later identified substituent group α;
   (xxxiii) a C₆₋₁₀ aryl group, (xxxiv) a heteroaryl group, (xxxv) a C₃₋₇ cycloalkyl group or (xxxvi) a heterocycloalkyl group,
   R^{E}, R^{F} and R^{G} independently represent a hydrogen atom, a cyano group, a carbamoyl group, a C₂₋₇ acyl group, a C₂₋₇ alkoxycarbonyl group, a C₆₋₁₀ aryl(C₂₋₇ alkoxycarbonyl) group, a nitro group, a C₁₋₆ alkylsulfonyl group, a sulfamoyl group, a carbamimidoyl group, or a C₁₋₆ alkyl group which may have any 1 to 5 substituents selected from the later identified substituent group β;
   or R^{E} and R^{F} bind together to form an ethylene group;
   or R^{F} and R^{G} bind together with the neighboring nitrogen atom to form an aliphatic cyclic amino group which may have any substituent selected from the later identified substituent group α;
   ring A represents a C₆₋₁₀ aryl group or a heteroaryl group;
   G represents a group represented by a formula: 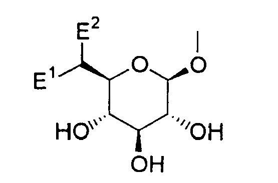 E¹ represents a hydrogen atom or a fluorine atom;
   E² represents a hydrogen atom, a fluorine atom or a methyl group;
   [substituent group α]
   a halogen atom, a hydroxy group, an amino group, a C₁₋₆ alkyl group, a C₁₋₆ alkoxy group, a halo(C₁₋₆ alkyl) group, a halo(C₁₋₆ alkoxy)group, a hydroxy(C₁₋₆ alkyl) group, a C₂₋₇ alkoxycarbonyl(C₁₋₆ alkyl) group, a hydroxy (C₁₋₆ alkoxy) group, an amino(C₁₋₆ alkyl) group, an amino (C₁₋₆ alkoxy) group, a mono or di(C₁₋₆ alkyl)amino group, a mono or di[hydroxy(C₁₋₆ alkyl)]amino group, a C₁₋₆ alkylsulfonyl group, a C₁₋₆ alkylsulfonylamino group, a C₁₋₆alkylsulfonylamino(C₁₋₆alkyl) group, a carboxy group, a C₂₋₇ alkoxycarbonyl group, a sulfamoyl group and -CON(R^{H})R^{I}
   [substituent group β]
   a halogen atom, a hydroxy group, an amino group, a C₁₋₆ alkoxy group, a C₁₋₆ alkylthio group, a halo (C₁₋₆ alkoxy) group, a halo(C₁₋₆ alkylthio) group, a hydroxy(C₁₋₆ alkoxy) group, a hydroxy(C₁₋₆ alkylthio) group, an amino(C₁₋₆ alkoxy) group, an amino(C₁₋₆ alkylthio) group, a mono or di(C₁₋₆ alkyl) amino group, a mono or di [hydroxy (C₁₋₆ alkyl)]amino group, an ureido group, a sulfamide group, a mono or di(C₁₋₆ alkyl) ureido group, a mono or di[hydroxy(C₁₋₆ alkyl)]ureido group, a mono or di(C₁₋₆ alkyl)sulfamide group, a mono or di[hydroxy(C₁₋₆ alkyl)]-sulfamide group, a C₂₋₇ acylamino group, an amino (C₂₋₇ acylamino) group, a C₁₋₆ alkylsulfonyl group, a C₁₋₆ alkylsulfonylamino group, a carbamoyl(C₁₋₆ alkylsulfonylamino) group, a carboxy group, a C₂₋₇ alkoxycarbonyl group, -CON(R^{H})R^{I}, and any of the following substituents (xxxvii) to (xxxxviii) which may have any 1 to 3 substituents selected from the above substituent group α on the ring;
   (xxxvii) a C₆₋₁₀ aryl group, (xxxviii) C₆₋₁₀ aryl-O-, (xxxix) a C₆₋₁₀ aryl (C₁₋₆ alkoxy) group, (xxxx) a C₆₋₁₀ aryl (C₁₋₆ alkylthio) group, (xxxxi) a heteroaryl group, (xxxxii) heteroaryl-O-, (xxxxiii) a C₃₋₇ cycloalkyl group, (xxxxiv) C₃₋₇ cycloalkyl-O-, (xxxxv) a heterocycloalkyl group, (xxxxvi) heterocycloalkyl-O-, (xxxxvii) an aliphatic cyclic amino group or (xxxxviii) an aromatic cyclic amino group,
   R^{H} and R^{I} independently represent a hydrogen atom or a C₁₋₆ alkyl group which may have any 1 to 3 substituents selected from the following substituent group γ;
   or both of R^{H} and R^{I} bind together with the neighboring nitrogen atom to form an aliphatic cyclic amino group which may have any 1 to 3 substituents selected from the following substituent group δ
   [substituent group γ]
   a halogen atom, a hydroxy group, an amino group, a C₁₋₆ alkoxy group, a halo (C₁₋₆ alkoxy) group, a hydroxy (C₁₋₆ alkoxy) group, an amino (C₁₋₆ alkoxy) group, a mono or di (C₁₋₆ alkyl) amino group, a mono or di [hydroxy (C₁₋₆ alkyl)] amino group, an ureido group, a sulfamide group, a mono or di (C₁₋₆ alkyl)ureido group, a mono or di [hydroxy (C₁₋₆ alkyl)] ureido group, a mono or di (C₁₋₆ alkyl)sulfamide group, a mono or di[hydroxy(C₁₋₆ alkyl)]-sulfamide group, a C₂₋₇ acylamino group, an amino (C₂₋₇ acylamino) group, a C₁₋₆ alkylsulfonyl group, a C₁₋₆ alkylsulfonylamino group, a carbamoyl(C₁₋₆ alkylsulfonylamino) group, a carboxy group, a C₂₋₇ alkoxycarbonyl group and -CON(R^{J})R^{K}
   [substituent group δ]
   a halogen atom, a hydroxy group, an amino group, a C₁₋₆ alkyl group, a C₁₋₆ alkoxy group, a halo(C₁₋₆ alkyl) group, a halo(C₁₋₆ alkoxy) group, a hydroxy(C₁₋₆ alkyl) group, a C₂₋₇ alkoxycarbonyl(C₁₋₆alkyl) group, a hydroxy(C₁₋₆alkoxy) group, an amino (C₁₋₆ alkyl) group, an amino(C₁₋₆ alkoxy) group, a mono or di(C₁₋₆ alkyl)amino group, a mono or di[hydroxy(C₁₋₆ alkyl)]amino group, a C₁₋₆ alkylsulfonyl group, a C₁₋₆ alkylsulfonylamino group, a C₁₋₆alkylsulfonylamino(C₁₋₆alkyl) group, a carboxy group, a C₂₋₇ alkoxycarbonyl group, a sulfamoyl group and -CON(R^{J})R^{K},
   R^{J} and R^{K} independently represent a hydrogen atom or a C₁₋₆ alkyl group which may have any 1 to 3 substituents selected from a hydroxy group, an amino group, a mono or di (C₁₋₆ alkyl) amino group, a C₂₋₇ alkoxycarbonyl group and a carbamoyl group;
   or both of R^{J} and R^{K} bind together with the neighboring nitrogen atom to form an aliphatic cyclic amino group which may have any 1 to 3 substituents selected from a hydroxy group, an amino group, a mono or di(C₁₋₆ alkyl) amino group, a C₁₋₆ alkyl group, a hydroxy (C₁₋₆ alkyl) group, a C₂₋₇ alkoxycarbonyl group, a C₂₋₇ alkoxycarbonyl(C₁₋₆ alkyl) group and a carbamoyl group, or a pharmaceutically acceptable salt thereof, or a prodrug thereof;
[2] a phenol derivative as described in the above [1], wherein G represents a β-D-glucopyranosyl group, or a pharmaceutically acceptable salt thereof, or a prodrug thereof;
[3] a phenol derivative as described in the above [1] or [2], wherein R³ and R⁴ independently represent a hydrogen atom, a hydroxy group, a halogen atom, a C₁₋₆ alkyl group, a C₂₋₆ alkenyl group, a C₂₋₆ alkynyl group, a C₁₋₆ alkoxy group, a C₂₋₆ alkenyloxy group, a C₁₋₆ alkylthio group, a C₂₋₆ alkenylthio group, a halo(C₁₋₆ alkyl) group, a halo(C₁₋₆ alkoxy) group, a halo(C₁₋₆ alkylthio) group, a hydroxy(C₁₋₆ alkyl) group, a hydroxy(C₂₋₆ alkenyl) group, a hydroxy (C₁₋₆ alkoxy) group or a hydroxy (C₁₋₆ alkylthio) group, or a pharmaceutically acceptable salt thereof, or a prodrug thereof;
[4] a phenol derivative as described in any one of the above [1] to [3], wherein the ring A represents a benzene ring or a pyridine ring, or a pharmaceutically acceptable salt thereof, or a prodrug thereof;
[5] a pharmaceutical composition comprising as an active ingredient a phenol derivative as described in any one of the above [1] to [4], or a pharmaceutically acceptable salt thereof, or a prodrug thereof;
[6] a human SGLT inhibitor comprising as an active ingredient a phenol derivative as described in any one of the above [1] to [4], or a pharmaceutically acceptable salt thereof, or a prodrug thereof;
[7] a human SGLT inhibitor as described in the above [6], wherein the SGLT is SGLT1 and/or SGLT2;
[8] a pharmaceutical composition as described in the above [5], which is an agent for the inhibition of postprandial hyperglycemia;
[9] a pharmaceutical composition as described in the above [5], which is an agent for the prevention or treatment of a disease associated with hyperglycemia;
[10] a pharmaceutical composition as described in the above [9], wherein the disease associated with hyperglycemia is a disease selected from the group consisting of diabetes, impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout;
[11] a pharmaceutical composition as described in the above [5], which is an agent for the inhibition of advancing impaired glucose tolerance into diabetes in a subject;
[12] a pharmaceutical composition as described in the above [5], wherein the dosage form is sustained release formulation;
[13] a human SGLT inhibitor as described in the above [6], wherein the dosage form is sustained release formulation;
[14] a method for the inhibition of postprandial hyperglycemia, which comprises administering an effective amount of a phenol derivative as described in any one of the above [1] to [4], or a pharmaceutically acceptable salt thereof, or a prodrug thereof;
[15] a method for the prevention or treatment of a disease associated with hyperglycemia, which comprises administering an effective amount of a phenol derivative as described in any one of the above [1] to [4], or a pharmaceutically acceptable salt thereof, or a prodrug thereof;
[16] a method for the prevention or treatment as described in the above [15], wherein the disease associated with hyperglycemia is a disease selected from the group consisting of diabetes,impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout;
[17] a method for the inhibition of advancing impaired glucose tolerance into diabetes in a subject, which comprises administering an effective amount of a phenol derivative as described in any one of the above [1] to [4], or a pharmaceutically acceptable salt thereof, or a prodrug thereof;
[18] a use of a phenol derivative as described in any one of the above [1] to [4], or a pharmaceutically acceptable salt thereof, or a prodrug thereof for the manufacture of a pharmaceutical composition for the inhibition of postprandial hyperglycemia;
[19] a use of a phenol derivative as described in any one of the above [1] to [4], or a pharmaceutically acceptable salt thereof, or a prodrug thereof for the manufacture of a pharmaceutical composition for the prevention or treatment of a disease associated with hyperglycemia;
[20] a use as described in the above [19], wherein the disease associated with hyperglycemia is a disease selected from the group consisting of diabetes, impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout;
[21] a use of a phenol derivative as described in any one of the above [1] to [4], or a pharmaceutically acceptable salt thereof, or a prodrug thereof for the manufacture of a pharmaceutical composition for the inhibition of advancing impaired glucose tolerance into diabetes in a subject;
[22] a pharmaceutical composition as described in the above [5], which comprises combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer;
[23] a human SGLT inhibitor as described in the above [6], which comprises combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer;
[24] a method for the inhibition of postprandial hyperglycemia as described in the above [14], which comprises administering in combination with at least one member selected from the group consisting of an insulin sensitivity enhancer, a glucose absorption inhibitor, abiguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesisinhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer;
[25] a method for the prevention or treatment of a disease associated with hyperglycemia as described in the above [15], which comprises administering in combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer;
[26] a method for the inhibition of advancing impaired glucose tolerance into diabetes in a subject as described in the above [17], which comprises administering in combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinicacidderivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer;
[27] a use of (A) a phenol derivative as described in any one of the above [1] to [4], or a pharmaceutically acceptable salt thereof, or a prodrug thereof and (B) at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the inhibition of postprandial hyperglycemia;
[28] a use of (A) a phenol derivative as described in any one of the above [1] to [4], or a pharmaceutically acceptable salt thereof, or a prodrug thereof and (B) at least one member selected from the group consisting of an insulin sensitivity enhancer, a glucose absorption inhibitor, abiguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761,bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the prevention or treatment of a disease associated with hyperglycemia;
[29] a use of (A) a phenol derivative as described in any one of the above [1] to [4], or a pharmaceutically acceptable salt thereof, or a prodrug thereof and (B) at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the inhibition of advancing impaired glucose tolerance into diabetes in a subject; and the like.

In the present invention, the term "C₁₋₆ alkyl group" means a straight-chained or branched alkyl group having 1 to 6 carbon atoms such as a methyl group, an ethyl group, a propyl group, an isopropyl group, a butyl group, an isobutyl group, a *sec*-butyl group, a *tert*-butyl group, a pentyl group, an isopentyl group, a neopentyl group, a *tert*-pentyl group, a hexyl group or the like; the term "C₁₋₆ alkylene group" or "-C₁₋₆ alkylene-" means a straight-chained or branched alkylene group having 1 to 6 carbon atoms such as a methylene group, an ethylene group, a trimethylene group, a tetramethylene group, a propylene group, a 1,1-dimethylethylene group or the like; the term "-C₁₋₅ alkylene-" means a straight-chained or branched alkylene group having 1 to 5 carbon atoms such as a methylene group, an ethylene group, a trimethylene group, a tetramethylene group, a propylene group, a 1,1-dimethylethylene group or the like; and the term "-C₁₋₄ alkylene-" means a straight-chained or branched alkylene group having 1 to 4 carbon atoms such as a methylene group, an ethylene group, a trimethylene group, a tetramethylene group, a propylene group, a 1,1-dimethylethylene group or the like. The term "hydroxy(C₁₋₆ alkyl) group" means the above C₁₋₆ alkyl group substituted by a hydroxy group; the term "amino (C₁₋₆ alkyl) group" means the above C₁₋₆ alkyl group substituted by an amino group such as an aminomethyl group, a 2-aminoethyl group or the like; the term "cyano(C₁₋₆ alkyl) group" means the above C₁₋₆ alkyl group substituted by a cyano group; the term "carbamoyl (C₁₋₆ alkyl) group" means the above C₁₋₆ alkyl group substituted by a carbamoyl group; and the term "carboxy (C₁₋₆ alkyl) group" means the above C₁₋₆ alkyl group substituted by a carboxy group.

The term "C₁₋₆ alkoxy group" means a straight-chained or branched alkoxy group having 1 to 6 carbon atoms such as a methoxy group, an ethoxy group, a propoxy group, an isopropoxy group, a butoxy group, an isobutoxy group, a *sec*-butoxy group, a *tert*-butoxy group, a pentyloxy group, an isopentyloxy group, a neopentyloxy group, a *tert*-pentyloxy group, a hexyloxy group or the like; the term "hydroxy(C₁₋₆ alkoxy) group" means the above C₁₋₆ alkoxy group substituted by a hydroxy group; the term "carboxy (C₁₋₆ alkoxy) group" means the above C₁₋₆ alkoxy group substituted by a carboxy group; the term "amino(C₁₋₆ alkoxy) group" means the above C₁₋₆ alkoxy group substituted by an amino group; and the term "carbamoyl(C₁₋₆ alkoxy) group" means the above C₁₋₆ alkoxy group substituted by a carbamoyl group. The term "C₁₋₆ alkylthio group" means a straight-chained or branched alkylthio group having 1 to 6 carbon atoms such as a methylthio group, an ethylthio group, a propylthio group, an isopropylthio group, a butylthio group, an isobutylthio group, a *sec*-butylthio group, a *tert*-butylthio group, a pentylthio group, an isopentylthio group, a neopentylthio group, a *tert*-pentylthio group, a hexylthio group or the like; the term "hydroxy(C₁₋₆ alkylthio) group" means the above C₁₋₆ alkylthio group substituted by a hydroxy group; the term "carboxy (C₁₋₆ alkylthio) group" means the above C₁₋₆ alkylthio group substituted by a carboxy group; and the term "amino(C₁₋₆ alkylthio) group" means the above C₁₋₆ alkylthio group substituted by an amino group.

The term "C₂₋₆ alkenyl group" means a straight-chained or branched alkenyl group having 2 to 6 carbon atoms such as a vinyl group, an allyl group, a 1-propenyl group, an isopropenyl group, a 1-butenyl group, a 2-butenyl group, a 2-methylallyl group or the like; the term "C₂₋₆ alkenylene group" or "-C₂₋₆ alkenylene-" means a straight-chained or branched alkenylene group having 2 to 6 carbon atoms such as a vinylene group, a propenylene group or the like; the term "-C₂₋₅ alkenylene-" means a straight-chained or branched alkenylene group having 2 to 5 carbon atoms such as a vinylene group, a propenylene group or the like; the term "-C₂₋₄ alkenylene-" means a straight-chained or branched alkenylene group having 2 to 4 carbon atoms such as a vinylene group, a propenylene group or the like; the term "hydroxy(C₂₋₆ alkenyl) group" means the above C₂₋₆ alkenyl group substituted by a hydroxy group; the term "carboxy (C₂₋₆ alkenyl) group" means the above C₂₋₆ alkenyl group substituted by a carboxy group; the term "C₂₋₆ alkenyloxy group" means a straight-chained or branched alkenyloxy group having 2 to 6 carbon atoms such as a vinyloxy group, an allyloxy group, a 1-propenyloxy group, an isopropenyloxy group, a 1-butenyloxy group, a 2-butenyloxy group, a 2-methylallyloxy group or the like; the term "C₂₋₆ alkenylthio group" means a straight-chained or branched alkenylthio group having 2 to 6 carbon atoms such as a vinylthio group, an allylthio group, a 1-propenylthio group, an isopropenylthio group, a 1-butenylthio group, a 2-butenylthio group, a 2-methylallylthio group or the like; the term "C₂₋₆ alkynyl group" means a straight-chained or branched alkynyl group having 2 to 6 carbon atoms such as an ethynyl group, a 2-propynyl group or the like; the term "-C₂₋₆ alkynylene-" means a straight-chained or branched alkynylene group having 2 to 6 carbon atoms such as an ethynylene group, a propynylene group or the like; the term "-C₂₋₅ alkynylene-" means a straight-chained or branched alkynylene group having 2 to 5 carbon atoms such as an ethynylene group, a propynylene group or the like; and the term "-C₂₋₄ alkynylene-" means a straight-chained or branched alkynylene group having 2 to 4 carbon atoms such as an ethynylene group, a propynylene group or the like.

The term "mono or di(C₁₋₆ alkyl)amino group" means an amino group mono-substituted by the above C₁₋₆ alkyl group or di-substituted by the same or different C₁₋₆ alkyl groups as defined above; the term "mono or di (C₁₋₆ alkyl) amino (C₁₋₆ alkyl) group" means the above C₁₋₆ alkyl group substituted by the above mono or di(C₁₋₆ alkyl)amino group; the term "mono or di(C₁₋₆ alkyl) amino (C₁₋₆ alkoxy) group" means the above C₁₋₆ alkoxy group substituted by the above mono or di (C₁₋₆ alkyl) amino group; the term "mono or di [hydroxy(C₁₋₆ alkyl)] amino group" means an amino group mono-substituted by the above hydroxy(C₁₋₆ alkyl) group or di-substituted by any of the above hydroxy (C₁₋₆ alkyl) groups; the term "mono or di(C₁₋₆ alkyl)ureido group" means an ureido group mono-substituted by the above C₁₋₆ alkyl group or di-substituted by any of the above C₁₋₆ alkyl groups; the term "mono or di [hydroxy (C₁₋₆ alkyl)] ureido group" means an ureido group mono-substituted by the above hydroxy(C₁₋₆ alkyl) group or di-substituted by any of the above hydroxy (C₁₋₆ alkyl) groups; the term "mono or di (C₁₋₆ alkyl) sulfamide group" means a sulfamide group mono-substituted by the above C₁₋₆ alkyl group or di-substituted by any of the above C₁₋₆ alkyl groups; the term "mono or di[hydroxy(C₁₋₆ alkyl)]sulfamide group" means a sulfamide group mono-substituted by the above hydroxy(C₁₋₆ alkyl) group or di-substituted by any of the above hydroxy(C₁₋₆ alkyl) groups; the term "C₂₋₇ acyl group"means a straight-chained or branched acyl group having 2 to 7 carbon atoms such as an acetyl group, a propionyl group, a butyryl group, an isobutyryl group, a valeryl group, a pivaloyl group, a hexanoyl group or the like; the term "C₂₋₇ acylamino group" means an amino group substituted by the above C₂₋₇ acyl group; and the term "amino (C₂₋₇ acylamino) group" means the above C₂₋₇ acylamino group substituted by an amino group, such as a 2-aminoacetylamino group, a 3-aminopropionylamino group or the like. The term "C₁₋₆ alkylsulfinyl group" means a straight-chained or branched alkylsulfinyl group having 1 to 6 carbon atoms such as a methylsulfinyl group, an ethylsulfinyl group or the like; the term "C₁₋₆ alkylsulfonyl group" means a straight-chained or branched alkylsulfonyl group having 1 to 6 carbon atoms such as a methanesulfonyl group, an ethanesulfonyl group or the like; the term "C₁₋₆ alkylsulfonylamino group" means an amino group substituted by the above C₁₋₆alkylsulfonyl group; the term "carbamoyl (C₁₋₆ alkylsulfonylamino) group" means the above C₁₋₆ alkylsulfonylamino group substituted by a carbamoyl group, such as a carbamoylmethanesulfonylamino group or the like; and the term "C₁₋₆ alkylsulfonylamino (C₁₋₆ alkyl) group" means the above C₁₋₆ alkyl group substituted by the above C₁₋₆ alkylsulfonylamino group.

The term "halogen atom" means a fluorine atom, a chlorine atom, a bromine atom or an iodine atom; the term "halo(C₁₋₆ alkyl) group" means the above C₁₋₆ alkyl group substituted by any 1 to 3 halogen atoms as defined above; the term "halo(C₁₋₆ alkoxy) group" means the above C₁₋₆ alkoxy group substituted by any 1 to 3 halogen atoms as defined above; and the term "halo(C₁₋₆ alkylthio) group" means the above C₁₋₆ alkylthio group substituted by any 1 to 3 halogen atoms as defined above. The term "C₂₋₇ alkoxycarbonyl group" means a straight-chained or branched alkoxycarbonyl group having 2 to 7 carbon atoms such as a methoxycarbonyl group, an ethoxycarbonyl group, a propoxycarbonyl group, an isopropoxycarbonyl group, a butoxycarbonyl group, an isobutyloxycarbonyl group, a *sec*-butoxycarbonyl group, a *tert*-butoxycarbonyl group, a pentyloxycarbonyl group, an isopentyloxycarbonyl group, a neopentyloxycarbonyl group, a *tert*-pentyloxycarbonyl group, a hexyloxycarbonyl group or the like; the term "C₂₋₇ alkoxycarbonyl(C₁₋₆ alkyl) group" means the above C₁₋₆ alkyl group substituted by the above C₂₋₇ alkoxycarbonyl group; the term "C₂₋₇ alkoxycarbonyl (C₁₋₆ alkoxy) group" means the above C₁₋₆ alkoxy group substituted by the above C₂₋₇ alkoxycarbonyl group; the term "C₂₋₇ alkoxycarbonyl(C₁₋₆ alkylthio) group" means the above C₁₋₆ alkylthio group substituted by the above C₂₋₇ alkoxycarbonyl group; and the term "C₂₋₇ alkoxycarbonyl(C₂₋₆ alkenyl) group" means the above C₂₋₆ alkenyl group substituted by the above C₂₋₇ alkoxycarbonyl group.

The term "C₃₋₇ cycloalkyl group" or "C₃₋₇ cycloalkyl-" means a cyclopropyl group, a cyclobutyl group, a cyclopentyl group, a cyclohexyl group or a cycloheptyl group; the term "C₃₋₇ cycloalkyl(C₁₋₆ alkyl) group" means the above C₁₋₆ alkyl group substituted by the above C₃₋₇ cycloalkyl group; the term "C₃₋₇ cycloalkyl (C₁₋₆ alkoxy) group" means the above C₁₋₆ alkoxy group substituted by the above C₃₋₇ cycloalkyl group; and the term "C₃₋₇ cycloalkyl(C₁₋₆ alkylthio) group" means the above C₁₋₆ alkylthio group substituted by the above C₃₋₇ cycloalkyl group. The term "heterocycloalkyl group" or "heterocycloalkyl-" means a 3 to 7-membered aliphatic heterocyclic group containing any 1 or 2 hetero atoms other than the binding position selected from an oxygen atom, a sulfur atom and a nitrogen atom in the ring, which is derived from morpholine, thiomorpholine, tetrahydrofuran, tetrahydropyran, aziridine, azetidine, pyrrolidine, imidazolidine, oxazoline, piperidine, piperazine, pyrazolidine, pyrroline, imidazoline or the like, or a 5 or 6-membered aliphatic heterocyclic group containing any 1 or 2 hetero atoms in the ring other than the binding position selected from an oxygen atom, a sulfur atom and a nitrogen atom fused with a 6-membered ring, which is derived from indoline, isoindoline, tetrahydroindoline, tetrahydroisoindoline, hexahydroindoline, hexahydroisoindoline or the like. The term "heterocycloalkyl (C₁₋₆ alkyl) group" means the above C₁₋₆ alkyl group substituted by the above heterocycloalkyl group; the term "heterocycloalkyl (C₁₋₆alkoxy) group" means the above C₁₋₆ alkoxy group substituted by the above heterocycloalkyl group; and the term "heterocycloalkyl(C₁₋₆ alkylthio) group" means the above C₁₋₆ alkylthio group substituted by the above heterocycloalkyl group.

The term "C₆₋₁₀ aryl group" or "C₆₋₁₀ aryl-" means an aromatic cyclic hydrocarbon group having 6 or 10 carbon atoms such as a phenyl group, a naphthyl group or the like; the term "C₆₋₁₀ aryl(C₁₋₆ alkyl) group" means the above C₁₋₆ alkyl group substituted by the above C₆₋₁₀ aryl group; the term "C₆₋₁₀ aryl(C₁₋₆ alkoxy) group" means the above C₁₋₆ alkoxy group substituted by the above C₆₋₁₀ aryl group; and the term "C₆₋₁₀ aryl (C₁₋₆ alkylthio) group" means the above C₁₋₆ alkylthio group substituted by the above C₆₋₁₀ aryl group. The term "C₆₋₁₀ arylsulfonylamino group" means a sulfonylamino group having the above C₆₋₁₀ aryl group, such as a benzenesulfonylamino group or the like; the term "C₆₋₁₀ aryl(C₂₋₇ alkoxycarbonyl) group" means the above C₂₋₇ alkoxycarbonyl group substituted by the above C₆₋₁₀ aryl group; and the term "heteroaryl group" or "heteroaryl-" means a 5 or 6-membered aromaticheterocyclic group containing any 1 to 4 hetero atoms in the ring other than the binding position selected from an oxygen atom, a sulfur atom and a nitrogen atom, which is derived from thiazole, oxazole, isothiazole, isooxazole, pyridine, pyrimidine, pyrazine, pyridazine, furan, pyrrole, thiophene, imidazole, pyrazole, oxadiazole, thiodiazole, tetrazole, furazan or the like, or a 5 or 6-membered aromatic heterocyclic group containing any 1 to 4 hetero atoms in the ring other than the binding position selected from an oxygen atom, a sulfur atom and a nitrogen atom fused with a 6-membered ring, which is derived from indole, isoindole, benzofuran, isobenzofuran, benzothiophen, benzooxazole, benzothiazole, indazole, benzoimidazole, quinoline, isoquinoline, phthalazine, quinoxaline, quinazoline, cinnoline, indolizine, naphthyridine, pteridine or the like. The term "heteroaryl(C₁₋₆ alkyl) group" means the above C₁₋₆ alkyl group substituted by the above heteroaryl group; the term "heteroaryl (C₁₋₆ alkoxy) group" means the above C₁₋₆ alkoxy group substituted by the above heteroaryl group; and the term "heteroaryl(C₁₋₆alkylthio) group" means the above C₁₋₆alkylthio group substituted by the above heteroaryl group.

The term "aliphatic cyclic amino group" means a 5 or 6-membered aliphatic cyclic amino group which may contain one hetero atom other than the nitrogen atom at the binding position selected from an oxygen atom, a sulfur atom and nitrogen atom in the ring, such as a morpholino group, a thiomorpholino group, a 1-aziridinyl group, a 1-azetidinyl group, a 1-pyrrolidinyl group, a piperidino group, a 1-imidazolidinyl group, a 1-piperazinyl group, a pyrazolidinyl group or the like; the term "aromatic cyclic amino group" means a 5-membered aromatic cyclic amino group which may contain 1 to 3 nitrogen atoms in the ring other than the nitrogen atom at the binding position, such as a 1-imidazolyl group, a 1-pyrrolyl group, a pyrazolyl group, a 1-tetrazolyl group or the like; the term "aromatic cyclic amino(C₁₋₆ alkyl) group" means the above C₁₋₆ alkyl group substituted by the above aromatic cyclic amino group; the term "aromatic cyclic amino(C₁₋₆ alkoxy) group" means the above C₁₋₆ alkoxy group substituted by the above aromatic cyclic amino group; and the term "aromatic cyclic amino (C₁₋₆ alkylthio) group" means the above C₁₋₆ alkylthio group substituted by the above aromatic cyclic amino group.

The term "hydroxy-protective group" means a hydroxy-protective group used in general organic synthesis such as a methyl group, a benzyl group, a methoxymethyl group, an acetyl group, a pivaloyl group, a benzoyl group, a *tert*-butyldimethylsilyl group, a *tert*-butyldiphenylsilyl group, an allyl group, a triphenylmethyl group or the like; the term "amino-protective group" means an amino-protective group used in general organic synthesis such as a benzyloxycarbonyl group, a *tert*-butoxycarbonyl group, a benzyl group, an acetyl group, a trifluoroacetyl group or the like; and the term "carboxy-protective group" means a carboxy-protective group used in general organic synthesis such as a methyl group, an ethyl group, a benzyl group, a *tert*-butyldimethylsilyl group, an allyl group or the like. In addition, in the substituent Q, the left-hand bond means a bond bound to a naphthalene ring and the right-hand bond means a bond bound to a ring A.

The compounds represented by the above general formula (I) of the present invention can be prepared according to the following procedures or analogous procedures thereof, or other procedures described in literatures or analogous procedures thereof.

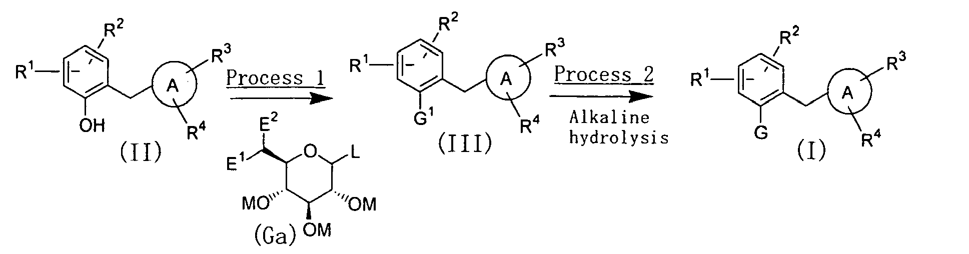

In the formula, G¹ represents the above G wherein any hydroxy group is protected; L represents a leaving group such as a trichloroacetoimidoyloxy group, an acetyloxy group or the like; M represents a hydroxy-protective group such as an acetyl group, a pivaloyl group, a benzoyl group or the like; R¹ to R⁴, E¹, E², G and ring A have the same meanings as defined above and with the proviso that in the case that there are a hydroxy group, an amino group and/or a carboxy group in each compound, a compound having a protective group can be suitably used.

### Process 1

A glycoside represented by the above general formula (III) can be prepared by subjecting a compound represented by the above general formula (II) to glycosidation using a sugar donor represented by the above general formula (Ga) in the presence of an activating agent such as boron trifluoride-diethyl ether complex, silver trifluoromethanesulfonate, tin (II) chloride, trimethylsilyl trifluoromethanesulfonate or the like in an inert solvent. As the solvent used, for example, dichloromethane, toluene, acetonitrile, nitromethane, ethyl acetate, diethyl ether, chloroform, a mixed solvent there of and the like can be illustrated. The reaction temperature is usually from -30°C to reflux temperature, and the reaction time is usually from 10 minutes to 1 day, varying based on a used starting material, solvent and reaction temperature.

### Process 2

A compound represented by the above general formula (I) of the present invention can be prepared by subjecting a glycoside represented by the above general formula (III) to alkaline hydrolysis to remove the protective group. As the solvent used, for example, water, methanol, ethanol, tetrahydrofuran, a mixed solvent thereof and the like can be illustrated. As a basic substance, for example, sodium hydroxide, sodium methoxide, sodium ethoxide or the like can be used. The treatment temperature is usually from 0°C to reflux temperature, and the treatment time is usually from 30 minutes to 1 day, varying based on a used starting material, solvent and treatment temperature.

The starting materials used in the above manufacturing methods can be prepared according to procedures described in literatures or analogous procedures thereof (for example, International publication WO01/68660, WO02/28872, WO02/44192, WO02/064606, WO03/011880 and WO01/74834 pamphlets).

The compounds represented by the above general formula (I) of the present invention obtained by the above production processes can be isolated andpurifiedby conventional separation means such as fractional recrystallization, purification using chromatography, solvent extraction and solid phase extraction.

The phenol derivatives represented by the above general formula (I) of the present invention can be converted into their pharmaceutically acceptable salts in the usual way. Examples of such salts include acid addition salts with mineral acids such as hydrochloric acid, hydrobromic acid, hydroiodic acid, sulfuric acid, nitric acid, phosphoric acid and the like, acid addition salts with organic acids such as formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like, salts with inorganic bases such as a sodium salt, a potassium salt and the like, and salts with organic bases such as N-methyl-D-glucamine, N,N'-dibenzyletylenediamine, 2-aminoethanol, tris(hydroxymethyl)aminomethane, arginine, lysine and the like.

The compounds represented by the above general formula (I) of the present invention include their solvates with pharmaceutically acceptable solvents such as water and ethanol.

Of the phenol derivatives represented by the above general formula (I) of the present invention and the prodrugs thereof, there are two geometrical isomers, cis(Z)-isomer and trans (E) -isomer, in each compound having an unsaturated bond. In the present invention, either of the isomers can be employed.

Of the phenol derivatives represented by the above general formula (I) of the present invention and the prodrugs thereof, there are two optical isomers, R-isomer and S-isomer, in each compound having an asymmetric carbon atom excluding the sugar moiety. In the present invention, either of the optical isomers can be employed, and a mixture of both optical isomers can be also employed.

A prodrug of a compound represented by the above general formula (I) of the present invention can be prepared by introducing an appropriate group forming a prodrug into any one or more groups selected from a hydroxy group and an amino group of the compound represented by the above general formula (I) using a corresponding reagent to produce a prodrug such as a halide compound or the like in the usual way, and then by suitably isolating and purificating in the usual way as occasion demands. As a group forming a prodrug used in a hydroxy group or an amino group, for example, a C₂₋₇ acyl group, a C₁₋₆ alkoxy(C₂₋₇ acyl) group, a C₂₋₇ alkoxycarbonyl(C₂₋₇ acyl) group, a C₂₋₇ alkoxycarbonyl group, a C₁₋₆ alkoxy(C₂₋₇ alkoxycarbonyl) group or the like can be illustrated. The term "C₁₋₆ alkoxy (C₂₋₇ acyl) group" means the above C₂₋₇ acyl group substituted by the above C₁₋₆ alkoxy group; the term "C₂₋₇ alkoxycarbonyl (C₂₋₇ acyl) group" means the above C₂₋₇ acyl group substituted by the above C₂₋₇ alkoxycarbonyl group; the term "C₁₋₆ alkoxy (C₂₋₇ alkoxycarbonyl) group" means the above C₂₋₇ alkoxycarbonyl group substituted by the above C₁₋₆ alkoxy group. In addition, as a group forming a prodrug, a glucopyranosyl group or a galactopyranosyl group can be illustrated. For example, these groups are preferably introduced into the hydroxy group at the 4 or 6-position of the glucopyranosyloxy group or the galactopyranosyloxy group, and are more preferably introduced into the hydroxy group at the 4 or 6-position of the glucopyranosyloxy group.

The phenol derivatives represented by the above general formula (I) of the present invention showed, for example, a potent inhibitory activity on human SGLT1 or SGLT2 in assay for inhibitory effects on human SGLT1 or SGLT2 activity as described below. Therefore, a naphthalene derivative represented by the above general formula (I) of the present invention can exert an excellent inhibitory activity of SGLT1 at the small intestine or an excellent inhibitory activity of SGLT2 at the kidney, and significantly inhibit blood glucose level increase or significantly lower blood glucose level. Therefore, a naphthalene derivative represented by the above general formula (I) of the present invention, a pharmaceutically acceptable salt thereof and a prodrug thereof is extremely useful as an agent for the inhibition of postprandial hyperglycemia, the inhibition of advancing into diabetes in a subject with impaired glucose tolerance and the prevention or treatment of a disease associated with hyperglycemia such as diabetes, impaired glucose tolerance (IGT), diabetic complications (e.g., retinopathy, neuropathy, nephropathy, ulcer, macroangiopathy), obesity, hyperinsulinemia, hyperlipidemia, hypercholesterolemia, hypertriglyceridemia, lipid metabolism disorder, atherosclerosis, hypertension, congestive heart failure, edema, hyperuricemia, gout or the like, which relates to SGLT1 activity at the small intestine and SGLT2 activity at the kidney.

Furthermore, the compounds of the present invention can be suitably used in combination with at least one member selected from the following drugs. Examples of the drugs which can be used in combination with the compounds of the present invention include an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor (PDGF), a platelet-derived growth factor (PDGF) analogue (e.g., PDGF-AA, PDGF-BB, PDGF-AB), epidermal growth factor (EGF), nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyltransferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

In case of uses of the compound of the present invention in combination with the above one or more drugs, the present invention includes either dosage forms of simultaneous administration as a single preparation or separated preparations in way of the same or different administration route, and administration at different dosage intervals as separated preparations in way of the same or different administration route. A pharmaceutical combination comprising the compound of the present invention and the above drug(s) includes both dosage forms as a single preparation and separated preparations for combination as mentioned above.

The compounds of the present invention can obtain more advantageous effects than additive effects in the prevention or treatment of the above diseases when using suitably in combination with the above one or more drugs. Also, the administration dose can be decreased in comparison with administration of either drug alone, or adverse effects of coadministrated drugs can be avoided or declined.

Concrete compounds as the drugs used for combination and preferable diseases to be treated are exemplified as follows. However, the present invention is not limited thereto, and the concrete compounds include their free compounds, and their or other pharmaceutically acceptable salts.

As insulin sensitivity enhancers, peroxisome proliferator-activated receptor-γagonists such as troglitazone, pioglitazone hydrochloride, rosiglitazone maleate, sodium darglitazone, GI-262570, isaglitazone, LG-100641, NC-2100, T-174, DRF-2189, CLX-0921, CS-011, GW-1929, ciglitazone, sodium englitazone and NIP-221, peroxisome proliferator-activated receptor-α agonists such as GW-9578 and BM-170744, peroxisome proliferator-activated receptor-α/γ agonists such as GW-409544, KRP-297, NN-622, CLX-0940, LR-90, SB-219994, DRF-4158 and DRF-MDX8, retinoid X receptor agonists such as ALRT-268, AGN-4204, MX-6054, AGN-194204, LG-100754 and bexarotene, and other insulin sensitivity enhancers such as reglixane, ONO-5816, MBX-102, CRE-1625, FK-614, CLX-0901, CRE-1633, NN-2344, BM-13125, BM-501050, HQL-975, CLX-0900, MBX-668, MBX-675, S-15261, GW-544, AZ-242, LY-510929, AR-H049020 and GW-501516 are illustrated. Insulin sensitivity enhancers are used preferably for diabetes, impaired glucose tolerance, diabetic complications, obesity, hyperinsulinemia, hyperlipidemia, hypercholesterolemia, hypertriglyceridemia, lipid metabolism disorder or atherosclerosis, and more preferably for diabetes, impaired glucose tolerance or hyperinsulinemia because of improving the disturbance of insulin signal transduction in peripheral tissues and enhancing glucose uptake into the tissues from the blood, leading to lowering of blood glucose level.

As glucose absorption inhibitors, for example, α-glucosidase inhibitors such as acarbose, voglibose, miglitol, CKD-711, emiglitate, MDL-25,637, camiglibose and MDL-73,945, α-amylase inhibitors such as AZM-127, SGLT1 inhibitors described in pamphlets of International Publication Nos. WO02/098893, WO2004/014932, WO2004/018491, W02004/019958 and the like are illustrated. Glucose absorption inhibitors are used preferably for diabetes, impaired glucose tolerance, diabetic complications, obesity or hyperinsulinemia, and more preferably for impaired glucose tolerance because of inhibiting the gastrointestinal enzymatic digestion of carbohydrates contained in foods, and inhibiting or delaying the absorption of glucose into the body.

As biguanides, phenformin, buformin hydrochloride, met formin hydrochloride or the like are illustrated. Biguanides are used preferably for diabetes, impaired glucose tolerance, diabetic complications or hyperinsulinemia, and more preferably for diabetes, impaired glucose tolerance or hyperinsulinemia because of lowering blood glucose level by inhibitory effects on hepatic gluconeogenesis, accelerating effects on anaerobic glycolysis in tissues or improving effects on insulin resistance in peripheral tissues.

As insulin secretion enhancers, tolbutamide, chlorpropamide, tolazamide, acetohexamide, glyclopyramide, glyburide (glibenclamide), gliclazide, 1-butyl-3-metanilyl-urea, carbutamide, glibornuride, glipizide, gliquidone, glisoxepide, glybuthiazol, glybuzole, glyhexamide, sodium glymidine, glypinamide, phenbutamide, tolcyclamide, glimepiride, nateglinide, mitiglinide calcium hydrate, repaglinide or the like are illustrated. In addition, the insulin secretion enhancers include glucokinase activators such as RO-28-1675. Insulin secretion enhancers are used preferably for diabetes, impaired glucose tolerance or diabetic complications, and more preferably for diabetes or impaired glucose tolerance because of lowering blood glucose level by acting on pancreatic β-cells and enhancing the insulin secretion.

As SGLT2 inhibitors, T-1095 and compounds described in Japanese patent publications Nos. Hei10-237089 and 2001-288178, and International Publications Nos. WO01/16147, WO01/27128, WO01/68660, WO01/74834, WO01/74835, WO02/28872, WO02/36602, WO02/44192, WO02/53573, WO03/000712, WO03/020737 and the like are illustrated. SGLT2 inhibitors are used preferably for diabetes, impaired glucose tolerance, diabetic complications, obesity or hyperinsulinemia, and more preferably for diabetes, impaired glucose tolerance, obesity or hyperinsulinemia because of lowering blood glucose level by inhibiting the reabsorption of glucose at the kidney's proximal tubule.

As insulin or insulin analogues, human insulin, animal-derived insulin, human or animal-derived insulin analogues or the like are illustrated. These preparations are used preferably for diabetes, impaired glucose tolerance or diabetic complications, and more preferably for diabetes or impaired glucose tolerance.

As glucagon receptor antagonists, BAY-27-9955, NNC-92-1687 or the like are illustrated; as insulin receptor kinase stimulants, TER-17411, L-783281, KRX-613 or the like are illustrated; as tripeptidyl peptidase II inhibitors, UCL-1397 or the like are illustrated; as dipeptidyl peptidase IV inhibitors, NVP-DPP728A, TSL-225, P-32/98 or the like are illustrated; as protein tyrosine phosphatase 1B inhibitors, PTP-112, OC-86839, PNU-177496 or the like are illustrated; as glycogen phosphorylase inhibitors, NN-4201, CP-368296 or the like are illustrated; as fructose-bisphosphatase inhibitors, R-132917 or the like are illustrated; as pyruvate dehydrogenase inhibitors, AZD-7545 or the like are illustrated; as hepatic gluconeogenesis inhibitors, FR-225659 or the like are illustrated; as glucagon-like peptide-1 analogues, exendin-4, CJC-1131 or the like are illustrated; as glucagon-like peptide 1 agonists; AZM-134, LY-315902 or the like are illustrated; and as amylin, amylin analogues or amylin agonists, pramlintide acetate or the like are illustrated. These drugs, glucose-6-phosphatase inhibitors, D-chiroinsitol, glycogen synthase kinase-3 inhibitors and glucagon-like peptide-1 are used preferably for diabetes, impaired glucose tolerance, diabetic complications or hyperinsulinemia, and more preferably for diabetes or impaired glucose tolerance.

As aldose reductase inhibitors, ascorbyl gamolenate, tolrestat, epalrestat, ADN-138, BAL-ARI8, ZD-5522, ADN-311, GP-1447, IDD-598, fidarestat, sorbinil, ponalrestat, risarestat, zenarestat, minalrestat, methosorbinil, AL-1567, imirestat, M-16209, TAT, AD-5467, zopolrestat, AS-3201, NZ-314, SG-210, JTT-811, lindolrestat or the like are illustrated. Aldose reductase inhibitors are preferably used for diabetic complications because of inhibiting aldose reductase and lowering excessive intracellular accumulation of sorbitol in accelerated polyol pathway which are in continuous hyperglycemic condition in the tissues in diabetic complications.

As advanced glycation endproducts formation inhibitors, pyridoxamine, OPB-9195, ALT-946, ALT-711, pimagedine hydrochloride or the like are illustrated. Advanced glycation endproducts formation inhibitors are preferably used for diabetic complications because of inhibiting formation of advanced glycation endproducts which are accelerated in continuous hyperglycemic condition in diabetes and declining of cellular damage.

As protein kinase C inhibitors, LY-333531, midostaurin or the like are illustrated. Protein kinase C inhibitors are preferably used for diabetic complications because of inhibiting of protein kinase C activity which is accelerated in continuous hyperglycemic condition in diabetes.

As γ-aminobutyric acid receptor antagonists, topiramate or the like are illustrated; as sodium channel antagonists, mexiletine hydrochloride, oxcarbazepine or the like are illustrated; as transcrit factor NF-κB inhibitors, dexlipotam or the like are illustrated; as lipid peroxidase inhibitors, tirilazad mesylate or the like are illustrated; as *N*-acetylated-α-linked-acid-dipeptidase inhibitors, GPI-5693 or the like are illustrated; and as carnitine derivatives, carnitine, levacecarninehydrochloride, levocarnitinechloride, levocarnitine, ST-261 or the like are illustrated. These drugs, insulin-like growth factor-I, platelet-derived growth factor, platelet derived growth factor analogues, epidermal growth factor, nerve growth factor, uridine, 5-hydroxy-1-methyl-hydantoin, EGB-761, bimoclomol, sulodexide and Y-128 are preferably used for diabetic complications.

As antidiarrhoics or cathartics, polycarbophil calcium, albumin tannate, bismuth subnitrate or the like are illustrated. These drugs are preferably used for diarrhea, constipation or the like accompanying diabetes or the like.

As hydroxymethylglutaryl coenzyme A reductase inhibitors, sodium cerivastatin, sodium pravastatin, lovastatin, simvastatin, sodiumfluvastatin, atorvastatin calcium hydrate, SC-4-5355, SQ-33600, CP-83101, BB-476, L-669262, S-2468, DMP-565, U-20685, BAY-x-2678, BAY-10-2987, calcium pitavastatin, calcium rosuvastatin, colestolone, dalvastatin, acitemate, mevastatin, crilvastatin, BMS-180431, BMY-21950, glenvastatin, carvastatin, BMY-22089, bervastatin or the like are illustrated. Hydroxymethylglutaryl coenzyme A reductase inhibitors are used preferably for hyperlipidemia, hypercholesterolemia, hypertriglyceridemia, lipid metabolism disorder or atherosclerosis, and more preferably for hyperlipidemia, hypercholesterolemia or atherosclerosis because of lowering blood cholesterol level by inhibiting hydroxymethylglutaryl coenzyme A reductase.

As fibrates, bezafibrate, beclobrate, binifibrate, ciprofibrate, clinofibrate, clofibrate, aluminum clofibrate, clofibric acid, etofibrate, fenofibrate, gemfibrozil, nicofibrate, pirifibrate, ronifibrate, simfibrate, theofibrate, AHL-157 or the like are illustrated. Fibrates are used preferably for hyperinsulinemia, hyperlipidemia, hypercholesterolemia, hypertriglyceridemia, lipid metabolism disorder or atherosclerosis, and more preferably for hyperlipidemia, hypertriglyceridemia or atherosclerosis because of activating hepatic lipoprotein lipase and enhancing fatty acid oxidation, leading to lowering of blood triglyceride level.

As β₃-adrenoceptor agonists, BRL-28410, SR-58611A, ICI-198157, ZD-2079, BMS-194449, BRL-37344, CP-331679, CP-114271, L-750355, BMS-187413, SR-59062A, BMS-210285, LY-377604, SWR-0342SA, AZ-40140, SB-226552, D-7114, BRL-35135, FR-149175, BRL-26830A, CL-316243, AJ-9677, GW-427353, N-5984, GW-2696, YM178 or the like are illustrated. β₃-Adrenoceptor agonists are used preferably for obesity, hyperinsulinemia, hyperlipidemia, hypercholesterolemia, hypertriglyceridemia or lipid metabolism disorder, and more preferably for obesity or hyperinsulinemia because of stimulating β₃-adrenoceptor in adipose tissue and enhancing the fatty acid oxidation, leading to induction of energy expenditure.

As acyl-coenzyme A cholesterol acyltransferase inhibitors, NTE-122, MCC-147, PD-132301-2, DUP-129, U-73482, U-76807, RP-70676, P-06139, CP-113818, RP-73163, FR-129169, FY-038, EAB-309, KY-455, LS-3115, FR-145237, T-2591, J-104127, R-755, FCE-28654, YIC-C8-434, avasimibe, CI-976, RP-64477, F-1394, eldacimibe, CS-505, CL-283546, YM-17E, lecimibide, 447C88, YM-750, E-5324, KW-3033, HL-004, eflucimibe or the like are illustrated. Acyl-coenzyme A cholesterol acyltransferase inhibitors are used preferably for hyperlipidemia, hypercholesterolemia, hypertriglyceridemia or lipid metabolism disorder, and more preferably for hyperlipidemia or hypercholesterolemia because of lowering blood cholesterol level by inhibiting acyl-coenzyme A cholesterol acyltransferase.

As thyroid hormone receptor agonists, sodium liothyronine, sodium levothyroxine, KB-2611 or the like are illustrated; as cholesterol absorption inhibitors, ezetimibe, SCH-48461 or the like are illustrated; as lipase inhibitors, orlistat, ATL-962, AZM-131, RED-103004 or the like are illustrated; as carnitine palmitoyltransferase inhibitors, etomoxir or the like are illustrated; as squalene synthase inhibitors, SDZ-268-198, BMS-188494, A-87049, RPR-101821, ZD-9720, RPR-107393, ER-27856, TAK-475 or the like are illustrated; as nicotinic acid derivatives, nicotinic acid, nicotinamide, nicomol, niceritrol, acipimox, nicorandil or the like are illustrated; as bile acid sequestrants, colestyramine, colestilan, colesevelam hydrochloride, GT-102-279 or the like are illustrated; as sodium/bile acid cotransporter inhibitors, 264W94, S-8921, SD-5613 or the like are illustrated; and as cholesterol ester transfer protein inhibitors, PNU-107368E, SC-795, JTT-705, CP-529414 or the like are illustrated. These drugs, probcol, microsomal trigylceride transfer protein inhibitors, lipoxygenase inhibitors and low-density lipoprotein receptor enhancers are preferably used for hyperlipidemia, hypercholesterolemia, hypertriglyceridemia or lipid metabolism disorder.

As appetite suppressants, monoamine reuptake inhibitors, serotonin reuptake inhibitors, serotonin releasing stimulants, serotonin agonists (especially 5HT_{2C}-agonists), noradrenaline reuptake inhibitors, noradrenaline releasing stimulants, α₁-adrenoceptor agonists, β₂-adrenoceptor agonists, dopamine agonists, cannabinoid receptorantagonists, γ-aminobutyric acid receptor antagonists, H₃-histamine antagonists, L-histidine, leptin, leptin analogues, leptin receptor agonists, melanocortin receptor agonists (especially, MC3-R agonists, MC4-R agonists), α-melanocyte stimulating hormone, cocaine-and amphetamine-regulated transcript, mahogany protein, enterostatin agonists, calcitonin, calcitonin-gene-related peptide, bombesin, cholecystokinin agonists (especially CCK-A agonists), corticotropin-releasing hormone, corticotrophin-releasing hormone analogues, corticotropin-releasing hormone agonists, urocortin, somatostatin, somatostatin analogues, somatostatin receptor agonists, pituitary adenylate cyclase-activating peptide,brain-derived neurotrophicfactor, ciliary neurotrophic factor, thyrotropin-releasing hormone, neurotensin, sauvagine, neuropeptide Y antagonists, opioid peptide antagonists, galanin antagonists, melanin-concentrating hormone receptor antagonists, agouti-related protein inhibitors and orexin receptor antagonists are illustrated. Concretely, as monoamine reuptake inhibitors, mazindol or the like are illustrated; as serotonin reuptake inhibitors, dexfenfluramine hydrochloride, fenfluramine, sibutramine hydrochloride, fluvoxamine maleate, sertraline hydrochloride or the like are illustrated; as serotonin agonists, inotriptan, (+)-norfenfluramine or the like are illustrated; as noradrenaline reuptake inhibitors, bupropion, GW-320659 or the like are illustrated; as noradrenaline releasing stimulants, rolipram, YM-992 or the like are illustrated; as β₂-adrenoceptor agonists, amphetamine, dextroamphetamine, phentermine, benzphetamine, methamphetamine, phendimetrazine, phenmetrazine, diethylpropion, phenylpropanolamine, clobenzorex or the like are illustrated; as dopamine agonists, ER-230, doprexin, bromocriptine mesylate or the like are illustrated; as cannabinoid receptor antagonists, rimonabant or the like are illustrated; as γ-aminobutyric acid receptor antagonists, topiramate or the like are illustrated; as H₃-histamine antagonists, GT-2394 or the like are illustrated; as leptin, leptin analogues or leptin receptor agonists, LY-355101 or the like are illustrated; as cholecystokinin agonists (especially CCK-A agonists), SR-146131, SSR-125180, BP-3.200, A-71623, FPL-15849, GI-248573, GW-7178, GI-181771, GW-7854, A-71378 or the like are illustrated; and as neuropeptide Y antagonists, SR-120819-A, PD-160170, NGD-95-1, BIBP-3226, 1229-U-91, CGP-71683, BIBO-3304, CP-671906-01, J-115814 or the like are illustrated. Appetite suppressants are used preferably for diabetes, impaired glucose tolerance, diabetic complications, obesity, hyperlipidemia, hypercholesterolemia, hypertriglyceridemia, lipid metabolism disorder, atherosclerosis, hypertension, congestive heart failure, edema, hyperuricemia or gout, and more preferably for obesity because of stimulating or inhibiting the activities of intracerebral monoamines or bioactive peptides in central appetite regulatory system and suppressing the appetite, leading to reduction of energy intake.

As angiotensin-converting enzyme inhibitors, captopril, enalaprimaleate, alacepril, delaprilhydrochloride, ramipril, lisinopril, imidapril hydrochloride, benazepril hydrochloride, ceronapril monohydrate, cilazapril, sodium fosinopril, perindopril erbumine, calcium moveltipril, quinapril hydrochloride, spirapril hydrochloride, temocapril hydrochloride, trandolapril, calcium zofenopril, moexipril hydrochloride, rentiapril or the like are illustrated. Angiotensin-converting enzyme inhibitors are preferably used for diabetic complications or hypertension.

As neutral endopeptidase inhibitors, omapatrilat, MDL-100240, fasidotril, sampatrilat, GW-660511X, mixanpril, SA-7060, E-4030, SLV-306, ecadotril or the like are illustrated. Neutral endopeptidase inhibitors are preferably used for diabetic complications or hypertension.

As angiotensin II receptor antagonists, candesartan cilexetil, candesartan cilexetil/hydrochlorothiazide, potassium losartan, eprosartan mesylate, valsartan, telmisartan, irbesartan, EXP-3174, L-158809, EXP-3312, olmesartan, tasosartan, KT-3-671, GA-0113, RU-64276, EMD-90423, BR-9701 or the like are illustrated. Angiotensin II receptor antagonists are preferably used for diabetic complications or hypertension.

As endothelin-converting enzyme inhibitors, CGS-31447, CGS-35066, SM-19712 or the like are illustrated; as endothelin receptor antagonists, L-749805, TBC-3214, BMS-182874, BQ-610, TA-0201, SB-215355, PD-180988, sodiumsitaxsentan, BMS-193884, darusentan, TBC-3711, bosentan, sodium tezosentan, J-104132, YM-598, S-0139, SB-234551, RPR-118031A, ATZ-1993, RO-61-1790, ABT-546, enlasentan, BMS-207940 or the like are illustrated. These drugs are preferably used for diabetic complications or hypertension, and more preferably for hypertension.

As diuretic agents, chlorthalidone, metolazone, cyclopenthiazide, trichloromethiazide, hydrochlorothiazide, hydroflumethiazide, benzylhydrochlorothiazide, penflutizide, methyclothiazide, indapamide, tripamide, mefruside, azosemide, etacrynic acid, torasemide, piretanide, furosemide, bumetanide, meticrane, potassium canrenoate, spironolactone, triamterene, aminophylline, cicletanine hydrochloride, LLU-α, PNU-80873A, isosorbide, D-mannitol, D-sorbitol, fructose, glycerin, acetazolamide, methazolamide, FR-179544, OPC-31260, lixivaptan, conivaptan hydrochloride or the like are illustrated. Diuretic drugs are preferably used for diabetic complications, hypertension, congestive heart failure or edema, and more preferably for hypertension, congestive heart failure or edema because of reducing blood pressure or improving edema by increasing urinary excretion.

As calcium antagonists, aranidipine, efonidipine hydrochloride, nicardipine hydrochloride, barnidipine hydrochloride, benidipine hydrochloride, manidipine hydrochloride, cilnidipine, nisoldipine, nitrendipine, nifedipine, nilvadipine, felodipine, amlodipine besilate, pranidipine, lercanidipine hydrochloride, isradipine, elgodipine, azelnidipine, lacidipine, vatanidipine hydrochloride, lemildipine, diltiazem hydrochloride, clentiazem maleate, verapamil hydrochloride, S-verapamil, fasudil hydrochloride, bepridil hydrochloride, gallopamil hydrochloride or the like are illustrated; as vasodilating antihypertensive agents,indapamide,todralazine hydrochloride, hydralazine hydrochloride, cadralazine, budralazine or the like are illustrated; as sympathetic blocking agents, amosulalol hydrochloride, terazosin hydrochloride, bunazosin hydrochloride, prazosin hydrochloride, doxazosin mesylate, propranolol hydrochloride, atenolol, metoprolol tartrate, carvedilol, nipradilol, celiprolol hydrochloride, nebivolol, betaxolol hydrochloride, pindolol, tertatolol hydrochloride, bevantolol hydrochloride, timolol maleate, carteolol hydrochloride, bisoprolol hemifumarate, bopindolol malonate, nipradilol, penbutolol sulfate, acebutolol hydrochloride, tilisolol hydrochloride, nadolol, urapidil, indoramin or the like are illustrated; as centrally acting antihypertensive agents, reserpine or the like are illustrated; and as α₂-adrenoceptor agonists, clonidine hydrochloride, methyldopa, CHF-1035, guanabenz acetate, guanfacine hydrochloride, moxonidine, lofexidine, talipexole hydrochloride or the like are illustrated. These drugs are preferably used for hypertension.

As antiplatelets agents, ticlopidine hydrochloride, dipyridamole, cilostazol, ethyl icosapentate, sarpogrelate hydrochloride, dilazep dihydrochloride, trapidil, beraprost sodium, aspirin or the like are illustrated. Antiplatelets agents are preferably used for atherosclerosis or congestive heart failure.

As uric acid synthesis inhibitors, allopurinol, oxypurinol or the like are illustrated; as uricosuric agents, benzbromarone, probenecid or the like are illustrated; and as urinary alkalinizers, sodium hydrogen carbonate, potassium citrate, sodium citrate or the like are illustrated. These drugs are preferably used for hyperuricemia or gout.

In case of uses in combination with a compound of the present invention, for example, in the use for diabetes, the combination with at least one member of the group consisting of an insulin sensitivity enhancer, a glucose absorption inhibitor, a biguanide, an insulin secretion enhancer, a SGLT2 inhibitors, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist and an appetite suppressant is preferable; the combination with at least one member of the group consisting of an insulin sensitivity enhancer, a glucose absorption inhibitor, a biguanide, an insulin secretion enhancer, a SGLT2 inhibitors, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue and an amylin agonist is more preferable; and the combination with at least one member of the group consisting of an insulin sensitivity enhancer, a glucose absorption inhibitor, a biguanide, an insulin secretion enhancer, a SGLT2 inhibitor and an insulin or insulin analogue is most preferable. Similarly, in the use for diabetic complications, the combination with at least one member of the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, glycogen synthase kinase-3 inhibitors, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid antagonist, a sodium channel antagonist, a transcriptfactor NF-κB inhibitor, a lipid peroxidase inhibitor, an *N*-acetylated-α-linked-acid-dipeptidase inhibitor, insulin-like growth factor-I, platelet-derived growth factor, a platelet derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, an angiotensin-converting enzyme inhibitor, a neutral endopeptidase inhibitor, an angiotensin II receptor antagonist, an endothelin-converting enzyme inhibitor, an endothelin receptor antagonist and a diuretic agent is preferable; and the combination with at least one member of the group consisting of an aldose reductase inhibitor, an angiotensin-converting enzyme inhibitor, a neutral endopeptidase inhibitor and an angiotensin II receptor antagonist is more preferable. Furthermore, in the use for obesity, the combination with at least one member of the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, a β₃-adrenoceptor agonist and an appetite suppressant is preferable; and the combination with at least one member of the group consisting of a glucose absorption inhibitor, a SGLT2 inhibitor, a β₃-adrenoceptor agonist and an appetite suppressant is more preferable.

When the pharmaceutical compositions of the present invention are employed in the practical treatment, various dosage forms are used depending on their uses. As examples of the dosage forms, powders, granules, fine granules, dry syrups, tablets, capsules, injections, solutions, ointments, suppositories, poultices and the like are illustrated, which are orally or parenterally administered. The pharmaceutical compositions of the present invention also include sustained release formulation including gastrointestinal mucoadhesive formulation (e.g., International publications Nos. WO99/10010, WO99/26606, and Japanese patent publication No. 2001-2567).

These pharmaceutical compositions can be prepared by admixing with or by diluting and dissolving with an appropriate pharmaceutical additive such as excipients, disintegrators, binders, lubricants, diluents, buffers, isotonicities, antiseptics, moistening agents, emulsifiers, dispersing agents, stabilizing agents, dissolving aids and the like, and formulating the mixture in accordance with conventional methods. In case of the uses of the compound of the present invention in combination with other drug(s), they can be prepared by formulating each active ingredient together or individually in a similar manner as defined above.

When the pharmaceutical compositions of the present invention are employed in the practical treatment, the dosage of a compound represented by the above general formula (I), a pharmaceutically acceptable salt thereof or a prodrug thereof as the active ingredient is appropriately decided depending on the age, sex, body weight and degree of symptoms and treatment of each patient, which is approximately within the range of from 0.1 to 1,000 mg per day per adult human in the case of oral administration and approximately within the range of from 0.01 to 300 mg per day per adult human in the case of parenteral administration, and the daily dose can be divided into one to several doses per day and administered suitably. Also, in case of the uses of the compound of the present invention in combination with other drug(s), the dosage of the compound of the present invention can be decreased, depending on the dosage of the drug(s).

### Examples

The present invention is further illustrated in more detail by way of the following Examples and Test Examples. However, the present invention is not limited thereto.

### Example 1

### Process 1

### 2-(4-Methoxybenzyl)phenyl 6-O-triphenylmethyl-β-D-glucopyranoside

To a solution of 2-(4-methoxybenzyl)phenyl β-D-glucopyranoside (1.0 g), triethylamine (0.48 g) and 4-dimethylaminopyridine (0.033 g) in *N,N*-dimethylformamide (15 mL) was addedchlorotriphenylmethane (0.81g) at roomtemperature. The mixture was stirred at room temperature overnight and poured into water, and the mixture was extracted with ethyl acetate. The organic layer was washed with water and brine, and dried over anhydrous magnesium sulfate. The solvent was removed under reduced pressure, and the residue was purified by column chromatography on silica gel (eluent: dichloromethane to dichloromethane/methanol =20/1) to give the title compound (1.5 g).

### Process 2

### 2-(4-Methoxybenzyl)phenyl 2,3,4-tri-O-benzoyl-β-D-glucopyranoside

To a solution of 2-(4-methoxybenzyl)phenyl 6-*O*-triphenylmethyl-β-D-glucopyranoside (1.5 g) and pyridine (1.9 g) in dichloromethane (20 mL) was added benzoyl chloride (1.2 g) at room temperature, and the mixture was stirred at room temperature for 3 hours. Water was added to the reaction mixture, and the mixture was extracted with diethyl ether. The organic layer was washed with water and brine, and dried over anhydrous magnesium sulfate, and the solvent was removed under reduced pressure. The residue was dissolved in diethyl ether (5 mL). Formic acid (5 mL) was added to the solution, and the mixture was stirredat roomtemperature for 3 hours. The reactionmixture was poured into water, and the mixture was extracted with diethyl ether. The organic layer was washed with water three times and brine, and dried over anhydrous magnesium sulfate. The solvent was removed under reduced pressure, and the residue was purified by column chromatography on silica gel (eluent: *n*-hexane/ethyl acetate =3/1 -2/1) to give the title compound (0.36 g).

### Process 3

### 2-(4-Methoxybenzyl)phenyl 6-deoxy-6-fluoro-2,3,4-tri-O-benzoyl-β-D-glucopyranoside

To a solution of 2-(4-methoxybenzyl)phenyl 2,3,4-tribenzoyl-β-D-glucopyranoside (0.15g) in dichloromethane (10 mL) was added (diethylamino) sulfur trifluoride (0. 14 g) at -40°C. The reaction mixture was warmed to room temperature and stirred for 3 hours. The reaction mixture was purified directly by column chromatography on silica gel (eluent: *n*-hexane to *n*-hexane/ethyl acetate = 3/1) to give the title compound (0.062 g).
¹H-NMR (CD₃OD) δ ppm:
3.63 (3H, s), 3.65-3.80 (2H, m), 4.35-4.52 (1H, m), 4.52-4.75 (2H, m), 5.60-5.68 (1H, m), 5.70 (1H, d, J=8.0Hz), 5.77 (1H, dd, J=8.0, 9.6Hz), 6.08 (1H, t, J=9. 6Hz) , 6. 50-6. 60 (2H, m) , 6. 80-6. 90 (2H, m), 6.90-7.05 (2H, m), 7.10-7.26 (2H, m), 7.30-7.7.39 (4H, m), 7.40-7.45 (2H, m), 7.45-7.60 (3H, m), 7.75-7.83 (2H, m), 7.85-7.90 (2H, m), 7.92-7.98 (2H, m)

### Process 4

### 2-(4-Methoxybenzyl)phenyl 6-deoxy-6-fluoro-β-D-glucopyranoside

To a solution of 2-(4-methoxybenzyl)phenyl 6-deoxy-6-fluoro-2,3,4-tri-*O*-benzoyl-β-D-glucopyranoside (0.061 g) in methanol (1 mL) was added sodiummethoxide (28% methanol solution, 0.034 mL) at room temperature, and the mixture was stirred at 60°C for 2 hours. The reaction mixture was concentrated under reduced pressure, and the residue was purified by column chromatography on silica gel (eluent: dichloromethane/methanol = 20/1) to give the title compound (0.013 g).
¹H-NMR (CD₃OD) δ ppm:
3.30-3.52 (3H, m), 3.52-3.65 (1H, m), 3.74 (3H, s), 3.94 (1H, d, J=15.1Hz), 4.03 (1H, d, J=15.1Hz), 4.59 (1H, ddd, J=4.8, 10.2, 47.7Hz), 4.64 (1H, ddd, J=1.7, 10.2, 47.8Hz), 4.90-4.95 (1H, m), 6.75-6.85 (2H, m) , 6.85-6.95 (1H, m), 7.00-7.05 (1H, m), 7.05-7.20 (4H, m)

### Test Example 1

### Assay for inhibitory effects on human SGLT1 activity

### 1) Cloning and construction of the vector expressing human SGLT1

The cDNA library was prepared for PCR amplification by reverse transcription from total RNA deprived from human small intestine (Ori gene) using oligo-dT as a primer. Using this cDNA library as a template, the DNA fragment coding 1 to 2005 bp of human SGLT1 (ACCESSION: M24847), which was reported by Hediger et al., was amplified by PCR method and inserted into the multi-cloning site of pcDNA3.1(-) (Invitrogen). The DNA sequence inserted was perfectly matched to the previously reported sequence.

### 2) Establishment of cell line stably expressing human SGLT1

The expression vector of human SGLT1 was digested by Sca I into a linear DNA. The linear DNA was transfected into CHO-K1 cells by means of lipofection (Effectene Transfection Reagent: QIAGEN). Neomycin resistant cell lines were selected by culture in the medium containing G418 (1 mg/mL, LIFE TECHNOLOGIES), and then the activity against the uptake of methyl-α-D-glucopyranoside was measured by the method described below. The cell line, which showed the greatest uptake activity, was selected and designated as CS1-5-11D. CS1-5-11D cells were cultured in the presence of G418 at 200 µg/mL.

### 3) Measurement of the inhibitory activity against the uptake of methyl-α-D-glucopyranoside (α-MG)

CS1-5-11D cells are seeded into a 96-well culture plate at a density of 3 × 10⁴ cells/well and cultured for 2 days, and are used in the uptake assay. A mixture of non-labeled (Sigma) and ¹⁴C-labeled α-MG (Amersham Pharmacia Biotech) is added to the uptake buffer (pH 7.4; containing 140 mM sodium chloride, 2 mM potassium chloride, 1 mM calcium chloride, 1 mM magnesium chloride, 10 mM 2-[4-(2-hydroxyethyl)-1-piperazinyl]ethane sulfonic acid and 5 mM tris(hydroxymethyl)aminomethane) at the final concentration of 1 mM. A test compound is dissolved in dimethyl sulfoxide, and then appropriately diluted with distilled water. The test compound solution is added to the uptake buffer containing 1 mM α-MG, and designated as a measurement buffer. For the control group, the measurement buffer without any test compound is prepared. For measuring the basal uptake, a basal uptake measurement buffer which contains 140 mM choline chloride instead of sodium chloride is prepared. After removing the culture medium of CS1-5-11D cells, 180 µL of the pre-treatment buffer (the basal uptake buffer without α-MG) is added to each well and incubated at 37°C for 10 minutes. After repeating the same treatment, the pre-treatment buffer is removed. To each well is added 75 µL of the measurement buffer or the basal uptake buffer is added and incubated at 37°C for 1 hour. After removing the measurement buffer, cells are washed twice with 180 µL per well of the washing buffer (the basal uptake buffer containing 10 mM non-labeled α-MG). The cells are solubilized by 75 µL per well of 0.2 mol/L sodium hydroxide. The cell lysates are transferred into PicoPlates (Packard), and then added 150 µL of MicroScint-40 (Packard) and mixed. Radioactivity is measured by means of micro-scintillation counter TopCount (Packard). One hundred % is set to the difference between the uptake in the control group and the basal uptake, and the uptake of methyl α-D-glucopyranoside at each drug concentration are calculated. The drug concentration, at which 50% uptake of methyl α-D-glucopyranoside is inhibited (IC₅₀ value), can be calculated using logit plot.

### Test Example 2

### Assay for inhibitory effects on human SGLT2 activity

### 1) Cloning and construction of the vector expressing human SGLT2

The cDNA library was prepared for PCR amplification by reverse transcription from total RNA deprived from human kidney (Ori gene) using oligo-dT as a primer. Using this cDNA library as a template, the DNA fragment coding 2 to 2039 bp of human SGLT2 (ACCESSION: M95549, M95299), which was reported by R. G. Wells et al., was amplified by PCR method and inserted into the multi-cloning site of pcDNA3.1(-) (Invitrogen). The DNA sequence inserted was perfectly matched to the previously reported sequence.

### 2) Establishment of cell line stably expressing human SGLT2

The expression vector of human SGLT2 was digested by Sca I into a linear DNA. The linear DNA was transfected into CHO-K1 cells by means of lipofection (Effectene Transfection Reagent: QIAGEN). Neomycin resistant cell lines were selected by culture in the medium containing G418 (1 mg/mL, LIFE TECHNOLOGIES), and then the activity against the uptake of methyl-α-D-glucopyranoside was measured by the method described below. The cell line, which showed the greatest uptake activity, was selected and designated as CS2-5E. CS2-5E cells were cultured in the presence of G418 at 200 µg/mL.

### 3) Measurement of the inhibitory activity against the uptake of methyl-α-D-glucopyranoside (α-MG)

CS2-5E cells were seeded into a 96-well culture plate at a density of 3 × 10⁴ cells/well and cultured for 2 days, and were used in the uptake assay. A mixture of non-labeled (Sigma) and ¹⁴C-labeled α-MG (Amersham Pharmacia Biotech) was added to the uptake buffer (pH 7.4; containing 140 mM sodium chloride, 2 mM potassiumchloride, 1mM calcium chloride, 1mM magnesium chloride, 10 mM 2-[4-(2-hydroxyethyl)-1-piperazinyl]ethane sulfonic acid and 5 mM tris(hydroxymethyl)aminomethane) at the final concentration of 1 mM. A test compound was dissolved in dimethyl sulfoxide, and then appropriately diluted with distilled water. The test compound solution was added to the uptake buffer containing 1 mM α-MG, and designated as a measurement buffer. For the control group, the measurement buffer without any test compound was prepared. For measuring the basal uptake, a basal uptake measurement buffer which contains 140 mM choline chloride instead of sodium chloride was prepared. After removing the culture medium of CS1-5-11D cells, 180 µL of the pre-treatment buffer (the basal uptake buffer without α-MG) was added to each well and incubated at 37°C for 10 minutes. After repeating the same treatment, the pre-treatment buffer was removed. To each well was added 75 µL of the measurement buffer or the basal uptake buffer was added and incubated at 37°C for 1 hour. After removing the measurement buffer, cells were washed twice with 180 µL per well of the washing buffer (the basal uptake buffer containing 10 mM non-labeled α-MG). The cells were solubilized by 75 µL per well of 0.2 mol/L sodium hydroxide. The cell lysates were transferred into PicoPlates (Packard), and then added 150 µL of MicroScint-40 (Packard) and mixed. Radioactivity was measured by means of micro-scintillation counter TopCount (Packard). One hundred % was set to the difference between the uptake in the control group and the basal uptake, and the uptake of methyl α-D-glucopyranoside at each drug concentration were calculated. The drug concentration, at which 50% uptake of methyl α-D-glucopyranoside was inhibited (IC₅₀ value), was calculated using logit plot. The results are shown in Table 1.

**[Table 1]**

| Test compound | IC₅₀ value (nM) |
|---|---|
| Example 1 | 86 |

### Industrial Applicability

The phenol derivatives represented by the above general formula (I) of the present invention, pharmaceutically acceptable salts thereof and prodrugs thereof exert an inhibitory activity in human SGLT and can suppress increase of blood glucose level or lower blood glucose level by inhibiting absorption of carbohydrate such as glucose at the small intestine or by inhibiting reabsorption of glucose at the kidney. Therefore, the present invention can provide excellent agents for the prevention or treatment of a disease associated with hyperglycemia such as diabetes, postprandial hyperglycemia, impaired glucose tolerance, diabetic complications, obesity or the like.

## Claims

1. A phenol derivative represented by the following general formula (I): 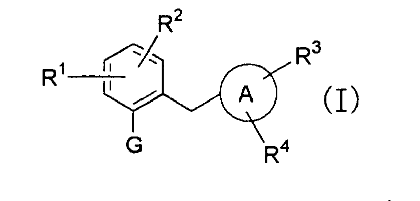 wherein
R¹ or R² independently represents a hydrogen atom, a hydroxy group, an amino group, a halogen atom, a C₁₋₆ alkyl group, a C₁₋₆ alkoxy group, a cyano group, a carboxy group, a C₂₋₇ alkoxycarbonyl group, a carbamoyl group, a mono or di(C₁₋₆ alkyl)amino group, a halo(C₁₋₆ alkyl) group, a hydroxy(C₁₋₆ alkyl) group, a cyano(C₁₋₆ alkyl) group, a carboxy(C₁₋₆ alkyl) group, a C₂₋₇ alkoxycarbonyl (C₁₋₆ alkyl) group, a carbamoyl (C₁₋₆ alkyl) group, an amino(C₁₋₆ alkyl) group, a mono or di(C₁₋₆ alkyl)amino(C₁₋₆ alkyl) group, a halo(C₁₋₆ alkoxy) group, a hydroxy (C₁₋₆ alkoxy) group, a carboxy (C₁₋₆ alkoxy) group, a C₂₋₇ alkoxycarbonyl(C₁₋₆ alkoxy) group, a carbamoyl(C₁₋₆ alkoxy) group, an amino(C₁₋₆ alkoxy) group, a mono or di(C₁₋₆ alkyl)amino(C₁₋₆ alkoxy) group, a C₃₋₇ cycloalkyl group, C₃₋₇ cycloalkyl-O-, a C₃₋₇ cycloalkyl(C₁₋₆ alkyl) group, or C₃₋₇ cycloalkyl(C₁₋₆ alkoxy) group;
R³ and R⁴ independently represent a hydrogen atom, a hydroxy group, a halogen atom, a C₁₋₆ alkyl group, a C₂₋₆ alkenyl group, a C₂₋₆ alkynyl group, a C₁₋₆ alkoxy group, a C₂₋₆ alkenyloxy group, a C₁₋₆ alkylthio group, a C₂₋₆ alkenylthio group, a halo(C₁₋₆ alkyl) group, a halo (C₁₋₆ alkoxy) group, a halo (C₁₋₆ alkylthio) group, a hydroxy(C₁₋₆alkyl) group,a hydroxy(C₂₋₆ alkenyl) group, a hydroxy (C₁₋₆ alkoxy) group, a hydroxy (C₁₋₆ alkylthio) group, a carboxy group, a carboxy(C₁₋₆ alkyl) group, a carboxy(C₂₋₆ alkenyl) group, a carboxy(C₁₋₆ alkoxy) group, a carboxy(C₁₋₆ alkylthio) group, a C₂₋₇ alkoxycarbonyl group, a C₂₋₇ alkoxycarbonyl(C₁₋₆ alkyl) group, a C₂₋₇ alkoxycarbonyl(C₂₋₆ alkenyl) group, a C₂₋₇ alkoxycarbonyl (C₁₋₆ alkoxy) group, a C₂₋₇ alkoxycarbonyl (C₁₋₆alkylthio) group, a C₁₋₆alkylsulfinyl group, a C₁₋₆ alkylsulfonyl group, -U-V-W-N(R⁵)-Z, or any of the following substituents (i) to (xxviii) which may have any 1 to 3 substituents selected from the later identified substituent group α on the ring;
(i) a C₆₋₁₀ aryl group, (ii) C₆₋₁₀ aryl-O-, (iii) C₆₋₁₀ aryl-S-, (iv) a C₆₋₁₀ aryl (C₁₋₆ alkyl) group, (v) a C₆₋₁₀ aryl (C₁₋₆ alkoxy) group, (vi) a C₆₋₁₀ aryl(C₁₋₆ alkylthio) group, (vii) a heteroaryl group, (viii) heteroaryl-O-, (ix) heteroaryl-S-, (x) a heteroaryl(C₁₋₆ alkyl) group, (xi) a heteroaryl (C₁₋₆ alkoxy) group, (xii) a heteroaryl (C₁₋₆ alkylthio) group, (xiii) a C₃₋₇ cycloalkyl group, (xiv) C₃₋₇ cycloalkyl-O-, (xv) C₃₋₇ cycloalkyl-S-, (xvi) a C₃₋₇ cycloalkyl (C₁₋₆ alkyl) group, (xvii) a C₃₋₇ cycloalkyl(C₁₋₆ alkoxy) group, (xviii) a C₃₋₇ cycloalkyl(C₁₋₆ alkylthio) group, (xix) a heterocycloalkyl group, (xx) heterocycloalkyl-O-, (xxi) heterocycloalkyl-S-, (xxii) a heterocycloalkyl(C₁₋₆ alkyl) group, (xxiii) a heterocycloalkyl(C₁₋₆ alkoxy) group, (xxiv) a heterocycloalkyl(C₁₋₆ alkylthio) group, (xxv) an aromatic cyclic amino group, (xxvi) an aromatic cyclic amino (C₁₋₆ alkyl) group, (xxvii) an aromatic cyclic amino(C₁₋₆ alkoxy) group or (xxviii) an aromatic cyclic amino(C₁₋₆ alkylthio) group,
U represents -O-, -S- or a single bond and with the proviso that at least one of V and W is not a single bond when U is -O- or -S-);
V represents a C₁₋₆ alkylene group which may have a hydroxy group, a C₂₋₆ alkenylene group or a single bond;
W represents -CO-, -SO₂-, -C(=NH)- or a single bond;
Z represents a hydrogen atom, a C₂₋₇ alkoxycarbonyl group, a C₆₋₁₀ aryl(C₂₋₇ alkoxycarbonyl) group, a formyl group, -R^{A}, -COR^{B}, -SO₂R^{B}, -CON(R^{C})R^{D}, -CSN(R^{C})R^{D}, -SO₂NHR^{A} or -C(=NR^{E})N(R^{F})R^{G};
R⁵, R^{A}, R^{C} and R^{D} independently represent a hydrogen atom, a C₁₋₆ alkyl group which may have any 1 to 5 substituents selected from the later identified substituent group β, or any of the following substituents (xxix) to (xxxii) which may have any 1 to 3 substituents selected from the later identified substituent group α;
(xxix) a C₆₋₁₀ aryl group, (xxx) a heteroaryl group, (xxxi) a C₃₋₇ cycloalkyl group or (xxxii) a heterocycloalkyl group
or Z and R⁵ bind together with the neighboring nitrogen atom to form an aliphatic cyclic amino group which may have any 1 to 3 substituents selected from the later identified substituent group α;
or R^{C} and R^{D} bind together with the neighboring nitrogen atom to form an aliphatic cyclic amino group which may have any 1 to 3 substituents selected from the later identified substituent group α;
R^{B} represents a C₂₋₇ alkoxycarbonyl group, a C₁₋₆ alkylsulfonylamino group, a C₆₋₁₀ arylsulfonylamino group, a C₁₋₆ alkyl group which may have any 1 to 5 substituents selected from the later identified substituent group β, or any of the following substituents (xxxiii) to (xxxvi) which may have any 1 to 3 substituents selected from the later identified substituent group α;
(xxxiii) a C₆₋₁₀ aryl group, (xxxiv) a heteroaryl group, (xxxv) a C₃₋₇ cycloalkyl group or (xxxvi) a heterocycloalkyl group,
R^{E}, R^{F} and R^{G} independently represent a hydrogen atom, a cyano group, a carbamoyl group, a C₂₋₇ acyl group, a C₂₋₇ alkoxycarbonyl group, a C₆₋₁₀ aryl (C₂₋₇ alkoxycarbonyl) group, a nitro group, a C₁₋₆ alkylsulfonyl group, a sulfamoyl group, a carbamimidoyl group, or a C₁₋₆ alkyl group which may have any 1 to 5 substituents selected from the later identified substituent group β;
or R^{E} and R^{F} bind together to form an ethylene group;
or R^{F} and R^{G} bind together with the neighboring nitrogen atom to form an aliphatic cyclic amino group which may have any substituent selected from the later identified substituent group α;
ring A represents a C₆₋₁₀ aryl group or a heteroaryl group;
G represents a group represented by a formula: 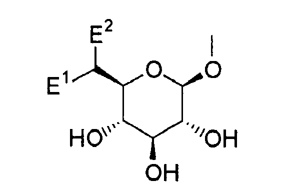 E¹ represents a hydrogen atom or a fluorine atom;
E² represents a hydrogen atom, a fluorine atom or
a methyl group;
[substituent group α]
a halogen atom, a hydroxy group, an amino group, a C₁₋₆ alkyl group, a C₁₋₆ alkoxy group, a halo(C₁₋₆ alkyl) group, a halo(C₁₋₆ alkoxy)group, a hydroxy(C₁₋₆ alkyl) group, a C₂₋₇ alkoxycarbonyl (C₁₋₆ alkyl) group, a hydroxy (C₁₋₆ alkoxy) group, an amino (C₁₋₆ alkyl) group, an amino (C₁₋₆ alkoxy) group, a mono or di(C₁₋₆ alkyl)amino group, a mono or di[hydroxy(C₁₋₆ alkyl)]amino group, a C₁₋₆ alkylsulfonyl group, a C₁₋₆ alkylsulfonylamino group, a C₁₋₆alkylsulfonylamino(C₁₋₆alkyl) group, a carboxy group, a C₂₋₇ alkoxycarbonyl group, a sulfamoyl group and -CON(R^{H})R^{I}
[substituent group β]
a halogen atom, a hydroxy group, an amino group, a C₁₋₆ alkoxy group, a C₁₋₆ alkylthio group, a halo (C₁₋₆ alkoxy) group, a halo(C₁₋₆ alkylthio) group, a hydroxy(C₁₋₆ alkoxy) group, a hydroxy(C₁₋₆ alkylthio) group, an amino (C₁₋₆ alkoxy) group, an amino (C₁₋₆alkylthio) group, a mono or di (C₁₋₆ alkyl) amino group, a mono or di[hydroxy(C₁₋₆ alkyl)]amino group, an ureido group, a sulfamide group, a mono or di(C₁₋₆ alkyl) ureido group, a mono or di[hydroxy(C₁₋₆ alkyl)]ureido group, a mono or di(C₁₋₆ alkyl)sulfamide group, a mono or di[hydroxy(C₁₋₆ alkyl)]-sulfamide group, a C₂₋₇ acylamino group, an amino (C₂₋₇ acylamino) group, a C₁₋₆ alkylsulfonyl group, a C₁₋₆ alkylsulfonylamino group, a carbamoyl(C₁₋₆ alkylsulfonylamino) group, a carboxy group, a C₂₋₇ alkoxycarbonyl group, -CON(R^{H})R^{I}, and any of the following substituents (xxxvii) to (xxxxviii) which may have any 1 to 3 substituents selected from the above substituent group α on the ring;
(xxxvii) a C₆₋₁₀ aryl group, (xxxviii) C₆₋₁₀ aryl-O-, (xxxix) a C₆₋₁₀ aryl (C₁₋₆ alkoxy) group, (xxxx) a C₆₋₁₀ aryl (C₁₋₆ alkylthio) group, (xxxxi) a heteroaryl group, (xxxxii) heteroaryl-O-, (xxxxiii) a C₃₋₇ cycloalkyl group, (xxxxiv) C₃₋₇ cycloalkyl-O-, (xxxxv) a heterocycloalkyl group, (xxxxvi) heterocycloalkyl-O-, (xxxxvii) an aliphatic cyclic amino group or (xxxxviii) an aromatic cyclic amino group,
R^{H} and R^{I} independently represent a hydrogen atom or a C₁₋₆ alkyl group which may have any 1 to 3 substituents selected from-the following substituent group γ;
or both of R^{H} and R^{I} bind together with the neighboring nitrogen atom to form an aliphatic cyclic amino group which may have any 1 to 3 substituents selected from the following substituent group δ
[substituent group γ]
a halogen atom, a hydroxy group, an amino group, a C₁₋₆ alkoxy group, a halo(C₁₋₆ alkoxy) group, a hydroxy(C₁₋₆ alkoxy) group, an amino(C₁₋₆ alkoxy) group, a mono or di(C₁₋₆ alkyl)amino group, a mono or di[hydroxy (C₁₋₆ alkyl)]amino group, an ureido group, a sulfamide group, a mono or di(C₁₋₆ alkyl) ureido group, a mono or di [hydroxy (C₁₋₆ alkyl) ] ureido group, a mono or di (C₁₋₆ alkyl)sulfamide group, a mono or di[hydroxy(C₁₋₆ alkyl)]-sulfamide group, a C₂₋₇ acylamino group, an amino (C₂₋₇ acylamino) group, a C₁₋₆ alkylsulfonyl group, a C₁₋₆ alkylsulfonylamino group, a carbamoyl(C₁₋₆ alkylsulfonylamino) group, a carboxy group, a C₂₋₇ alkoxycarbonyl group and -CON(R^{J})R^{K}
[substituent group δ]
a halogen atom, a hydroxy group, an amino group, a C₁₋₆ alkyl group, a C₁₋₆ alkoxy group, a halo(C₁₋₆ alkyl) group, a halo(C₁₋₆ alkoxy) group, a hydroxy(C₁₋₆ alkyl) group, a C₂₋₇ alkoxycarbonyl (C₁₋₆ alkyl) group, a hydroxy (C₁₋₆ alkoxy) group, an amino (C₁₋₆ alkyl) group, an amino (C₁₋₆ alkoxy) group, a mono or di(C₁₋₆ alkyl)amino group, a mono or di[hydroxy(C₁₋₆ alkyl)]amino group, a C₁₋₆ alkylsulfonyl group, a C₁₋₆ alkylsulfonylamino group, a C₁₋₆alkylsulfonylamino(C₁₋₆alkyl) group, a carboxy group, a C₂₋₇ alkoxycarbonyl group, a sulfamoyl group and -CON(R^{J})R^{K},
R^{J} and R^{K} independently represent a hydrogen atom or a C₁₋₆ alkyl group which may have any 1 to 3 substituents selected from a hydroxy group, an amino group, a mono or di (C₁₋₆ alkyl) amino group, a C₂₋₇ alkoxycarbonyl group and a carbamoyl group;
or both of R^{J} and R^{K} bind together with the neighboring nitrogen atom to form an aliphatic cyclic amino group which may have any 1 to 3 substituents selected from a hydroxy group, an amino group, a mono or di (C₁₋₆ alkyl) amino group, a C₁₋₆ alkyl group, a hydroxy(C₁₋₆ alkyl) group, a C₂₋₇ alkoxycarbonyl group, a C₂₋₇ alkoxycarbonyl (C₁₋₆ alkyl) group and a carbamoyl group, or a pharmaceutically acceptable salt thereof, or a prodrug thereof.

2. A phenol derivative as claimed in claim 1, wherein G represents a β-D-glucopyranosyl group, or a pharmaceutically acceptable salt thereof, or a prodrug thereof.

3. A phenol derivative as claimed in claim 1 or 2, wherein R³ and R⁴ independently represent a hydrogen atom, a hydroxy group, a halogen atom, a C₁₋₆ alkyl group, a C₂₋₆ alkenyl group, a C₂₋₆ alkynyl group, a C₁₋₆ alkoxy group, a C₂₋₆ alkenyloxy group, a C₁₋₆ alkylthio group, a C₂₋₆ alkenylthio group, a halo(C₁₋₆ alkyl) group, a halo (C₁₋₆ alkoxy) group, a halo (C₁₋₆ alkylthio) group, a hydroxy (C₁₋₆ alkyl) group, a hydroxy (C₂₋₆ alkenyl) group, a hydroxy(C₁₋₆alkoxy) group or a hydroxy (C₁₋₆ alkylthio) group, or a pharmaceutically acceptable salt thereof, or a prodrug thereof.

4. A phenol derivative as claimed in any one of claims 1 to 3, wherein the ring A represents a benzene ring or a pyridine ring, or a pharmaceutically acceptable salt thereof, or a prodrug thereof.

5. A pharmaceutical composition comprising as an active ingredient a phenol derivative as claimed in any one of claims 1 to 4, or a pharmaceutically acceptable salt thereof, or a prodrug thereof.

6. A human SGLT inhibitor comprising as an active ingredient a phenol derivative as claimed in any one of claims 1 to 4, or apharmaceuticallyacceptable salt thereof, or a prodrug thereof.

7. A human SGLT inhibitor as claimed in claim 6, wherein the SGLT is SGLT1 and/or SGLT2.

8. A pharmaceutical composition as claimed in claim 5, which is an agent for the inhibition of postprandial hyperglycemia.

9. A pharmaceutical composition as claimed in claim 5, which is an agent for the prevention or treatment of a disease associated with hyperglycemia.

10. A pharmaceutical composition as claimed in claim 9, wherein the disease associated with hyperglycemia is a disease selected from the group consisting of diabetes,impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout.

11. A pharmaceutical composition as claimed in claim 5, which is an agent for the inhibition of advancing impaired glucose tolerance into diabetes in a subject.

12. A pharmaceutical composition as claimed in claim 5, wherein the dosage form is sustained release formulation.

13. A human SGLT inhibitor as claimed in claim 6, wherein the dosage form is sustained release formulation.

14. A method for the inhibition of postprandial hyperglycemia, which comprises administering an effective amount of a phenol derivative as claimed in any one of claims 1 to 4, or a pharmaceutically acceptable salt thereof, or a prodrug thereof.

15. A method for the prevention or treatment of a disease associated with hyperglycemia, which comprises administering an effective amount of a phenol derivative as claimed in any one of claims 1 to 4, or a pharmaceutically acceptable salt thereof, or a prodrug thereof.

16. A method for the prevention or treatment as claimed in claim 15, wherein the disease associated with hyperglycemia is a disease selected from the group consisting of diabetes, impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout.

17. A method for the inhibition of advancing impaired glucose tolerance into diabetes in a subject, which comprises administering an effective amount of a phenol derivative as claimed in any one of claims 1 to 4, or a pharmaceutically acceptable salt thereof, or a prodrug thereof.

18. A use of a phenol derivative as claimed in any one of claims 1 to 4, or a pharmaceutically acceptable salt thereof, or a prodrug thereof for the manufacture of a pharmaceutical composition for the inhibition of postprandial hyperglycemia.

19. A use of a phenol derivative as claimed in any one of claims 1 to 4, or a pharmaceutically acceptable salt thereof, or a prodrug thereof for the manufacture of a pharmaceutical composition for the prevention or treatment of a disease associated with hyperglycemia.

20. A use as claimed in claim 19, wherein the disease associated with hyperglycemia is a disease selected from the group consisting of diabetes, impaired glucose tolerance, diabetic complications, obesity, hyperinsulinemia, hyperlipidemia, hypercholesterolemia, hypertriglyceridemia, lipid metabolism disorder, atherosclerosis, hypertension, congestive heart failure, edema, hyperuricemia and gout.

21. A use of a phenol derivative as claimed in any one of claims 1 to 4, or a pharmaceutically acceptable salt thereof, or a prodrug thereof for the manufacture of a pharmaceutical composition for the inhibition of advancing impaired glucose tolerance into diabetes in a subject.

22. A pharmaceutical composition as claimed in claim 5, which comprises combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesisinhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

23. A human SGLT inhibitor as claimed in claim 6, which comprises combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

24. A method for the inhibition of postprandial hyperglycemia as claimed in claim 14, which comprises administering in combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, abileacidsequestrant, asodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

25. A method for the prevention or treatment of a disease associated with hyperglycemia as claimed in claim 15, which comprises administering in combination with at least one member selected from the group consisting of an insulin sensitivity enhancer, a glucose absorption inhibitor, abiguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesisinhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

26. A method for the inhibition of advancing impaired glucose tolerance into diabetes in a subject as claimed in claim 17, which comprises administering in combination with at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesisinhibitor, D-chiroinsitol, a glycogen synthase kinase-3 inhibitor, glucagon-likepeptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer.

27. A use of (A) a phenol derivative as claimed in any one of claims 1 to 4, or a pharmaceutically acceptable salt thereof, or a prodrug thereof and (B) at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the inhibition of postprandial hyperglycemia.

28. A use of (A) a phenol derivative as claimed in any one of claims 1 to 4, or a pharmaceutically acceptable salt thereof, or a prodrug thereof and (B) at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesisinhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the prevention or treatment of a disease associated with hyperglycemia.

29. A use of (A) a phenol derivative as claimed in any one of claims 1 to 4, or a pharmaceutically acceptable salt thereof, or a prodrug thereof and (B) at least one member selected from the group consisting of an insulin sensitivity enhancer, a glucose absorption inhibitor, a biguanide, an insulin secretion enhancer, a SGLT2 inhibitor, an insulin or insulin analogue, a glucagon receptor antagonist, an insulin receptor kinase stimulant, a tripeptidyl peptidase II inhibitor, a dipeptidyl peptidase IV inhibitor, a protein tyrosine phosphatase-1B inhibitor, a glycogen phosphorylase inhibitor, a glucose-6-phosphatase inhibitor, a fructose-bisphosphatase inhibitor, a pyruvate dehydrogenase inhibitor, a hepatic gluconeogenesis inhibitor, D-chiroinsitol, a glycogen synthase kinase-3 inhibitor, glucagon-like peptide-1, a glucagon-like peptide-1 analogue, a glucagon-like peptide-1 agonist, amylin, an amylin analogue, an amylin agonist, an aldose reductase inhibitor, an advanced glycation endproducts formation inhibitor, a protein kinase C inhibitor, a γ-aminobutyric acid receptor antagonist, a sodium channel antagonist, a transcript factor NF-κB inhibitor, a lipid peroxidase inhibitor, an *N*-acetylated-α-linked-acid-dipeptidase inhibitor, insulin-like growth factor-I, platelet-derived growth factor, a platelet-derived growth factor analogue, epidermal growth factor, nerve growth factor, a carnitine derivative, uridine, 5-hydroxy-1-methylhydantoin, EGB-761, bimoclomol, sulodexide, Y-128, an antidiarrhoics, cathartics, a hydroxymethylglutaryl coenzyme A reductase inhibitor, a fibrate, a β₃-adrenoceptor agonist, an acyl-coenzyme A cholesterol acyltransferase inhibitor, probcol, a thyroid hormone receptor agonist, a cholesterol absorption inhibitor, a lipase inhibitor, a microsomal triglyceride transfer protein inhibitor, a lipoxygenase inhibitor, a carnitine palmitoyl-transferase inhibitor, a squalene synthase inhibitor, a low-density lipoprotein receptor enhancer, a nicotinic acid derivative, a bile acid sequestrant, a sodium/bile acid cotransporter inhibitor, a cholesterol ester transfer protein inhibitor, an appetite suppressant, an angiotensin-converting enzyme inhibitor, a neutral endopeptidase inhibitor, an angiotensin II receptor antagonist, an endothelin-converting enzyme inhibitor, an endothelin receptor antagonist, a diuretic agent, a calcium antagonist, a vasodilating antihypertensive agent, a sympathetic blocking agent, a centrally acting antihypertensive agent, an α₂-adrenoceptor agonist, an antiplatelets agent, a uric acid synthesis inhibitor, a uricosuric agent and a urinary alkalinizer, for the manufacture of a pharmaceutical composition for the inhibition of advancing impaired glucose tolerance into diabetes in a subject.
